(19) Europäisches Patentamt
European Patent Office
Office européen des brevets

(11) EP 2 512 409 B1

(12) EUROPÄISCHE PATENTSCHRIFT

(45) Veröffentlichungstag und Bekanntmachung des Hinweises auf die Patenterteilung:
**17.12.2014 Patentblatt 2014/51**

(21) Anmeldenummer: **10765778.5**

(22) Anmeldetag: **04.10.2010**

(51) Int Cl.:
***A61K 8/22*** *(2006.01)* ***A61K 8/49*** *(2006.01)*
***A61Q 5/08*** *(2006.01)*

(86) Internationale Anmeldenummer:
**PCT/EP2010/064720**

(87) Internationale Veröffentlichungsnummer:
**WO 2011/079974 (07.07.2011 Gazette 2011/27)**

(54) **PHTHALIMIDE ALS BLEICHAKTIVATOREN**

PHTHALIMIDES AS BLEACH ACTIVATORS

PHTHALIMIDES UTILISÉS COMME ACTIVATEURS DE DÉCOLORATION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **18.12.2009 DE 102009054949**

(43) Veröffentlichungstag der Anmeldung:
**24.10.2012 Patentblatt 2012/43**

(73) Patentinhaber: **Henkel AG & Co. KGaA**
**40589 Düsseldorf (DE)**

(72) Erfinder:
- **GROß, Wibke**
**41836 Hückelhoven (DE)**
- **NEMITZ, Ralph**
**41363 Jüchen (DE)**
- **KROOS, Astrid**
**40789 Monheim (DE)**

(56) Entgegenhaltungen:
**EP-A1- 1 161 936 DE-A1-102005 003 412**
**DE-B- 1 091 976 GB-A- 2 270 690**

EP 2 512 409 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

Printed by Jouve, 75001 PARIS (FR)

## Beschreibung

Die vorliegende Erfindung betrifft Mittel zum Aufhellen von keratinischen Fasern, insbesondere menschlicher Haare, die sich dadurch auszeichnen, dass die Mittel neben einem chemischen Oxidationsmittel mindestens ein bestimmtes kationisches Phthalimid enthalten. Durch deren Verwendung auf keratinischen Fasern wird die Aufhellleistung von Aufhell- und Blondiermitteln signifikant verbessert. Weiterhin betrifft die vorliegende Erfindung eine Mehrkomponentenverpackungseinheit (Kit-of-Parts) zur Aufhellung keratinischer Fasern, welche getrennt voneinander konfektioniert mindestens ein Mittel, enthaltend ein bestimmtes kationisches Phthalimid, eine Oxidationsmittelzubereitung und gegebenenfalls ein Blondierpulver umfasst.

Die Veränderung von Form und Farbe der Haare stellt einen wichtigen Bereich der modernen Kosmetik dar und erlaubt die Anpassung des Erscheinungsbilds der Haare sowohl an aktuelle Modeströmungen als auch an individuelle Wünsche der einzelnen Person. Dabei können Dauerwell- und andere die Haarform verändernde Verfahren nahezu unabhängig vom Typ der zu behandelnden Haare eingesetzt werden. Dagegen sind Färbe- und Blondierverfahren auf bestimmte Ausgangshaarfarben begrenzt.

Das Aufhellen der eigenen Haarfarbe ist seit jeher der Wunsch vieler Verbraucher, da eine blonde Haarfarbe als attraktiv und in modischer Hinsicht erstrebenswert betrachtet wird. Für diesen Zweck sind im Markt verschiedene Blondiermittel mit unterschiedlicher Blondierleistung erhältlich. Die in diesen Produkten enthaltenen Oxidationsmittel sind in der Lage, durch die oxidative Zerstörung des haareigenen Farbstoffes Melanin die Haarfaser aufzuhellen. Für einen moderaten Blondiereffekt genügt der Einsatz von Wasserstoffperoxid - gegebenenfalls unter Einsatz von Ammoniak oder anderen Alkalisierungsmitteln - als Oxidationsmittel allein, für das Erzielen eines stärkeren Blondiereffektes wird üblicherweise eine Mischung aus Wasserstoffperoxid und Peroxodisulfatsalzen und/oder Peroxomonosulfatsalzen eingesetzt.

Mit der Aufhellung geht jedoch auch eine Schädigung des Haares einher, da nicht nur die Farbstoffe des Haares, sondern auch die übrigen Strukturbestandteile des Haares oxidativ geschädigt werden. Je nach Ausprägung des Schädigungsgrades reicht dieser von rauem, sprödem und schwieriger auskämmbarem Haar über eine verminderte Widerstandsfähigkeit und Reißfestigkeit des Haares bis hin zu Haarbruch. Je größer die Menge des eingesetzten Wasserstoffperoxids und gegebenenfalls der Peroxodisulfate ist, desto stärkere Schädigungen werden in der Regel auf der Keratinfaser hervorgerufen. Haarfärbe- bzw. Aufhellmittel, welche eine gute Aufhellleistung zeigen, ohne gleichzeitig die Haarfaser zu schädigen, sind bislang nicht bekannt. Aufgabe dieser Erfindungsmeldung ist es daher, neuartige Mittel zum Aufhellen bzw. Blondieren von Haaren bereitzustellen, welche in ihrer Aufhellleistung den üblichen auf dem Markt befindlichen Mitteln vergleichbar oder überlegen sind, gleichzeitig jedoch eine verringerte Haarschädigung aufweisen. Bei Konsumenten mit sehr dunklem Haar ist selbst bei Anwendung von hohen Wasserstoffperoxidkonzentrationen in Kombination mit Persulfatsalzen keine Erzeugung von hellen Blondnuancen möglich. Auch wiederholte Anwendungen sind aufgrund zunehmender Haarschädigungen nicht durchführbar. Es ist daher weiterhin die Aufgabe dieser Erfindung, ein Mittel bereitzustellen, dessen Aufhellvermögen das der marktüblichen Mittel bestehend aus Wasserstoffperoxid und Peroxodisulfatsalzen (Natriumperoxodisulfat, Ammoniumperoxodisulfat und/oder Kaliumperoxodisulfat) übertrifft.

Nach EP 508623 A2 sind bestimmte kationische Phthalimidderivate in Form ihrer Percarbonsäuren bekannt, die in Gegenwart von Perboraten als Bleichmittel in der Textilbleiche eingesetzt werden. Der Einsatz der entsprechenden Phthalimide in Blondiermitteln zur Aufhellung von Haaren ist bislang nicht bekannt. Bei der Bleiche von Textilien und der Aufhellung von Haaren werden unterschiedliche, teilweise stark voneinander differierende Anwendungsparameter gewählt, so dass die Versuchsergebnisse des eines Anwendungsbereichs nicht auf den anderen übertragbar sind. So unterscheiden sich beispielsweise sowohl die Formulierung als auch die zu wählenden Temperaturen bei beiden Bleichprozessen stark. Aus dem Stand der Technik war daher nicht vorhersehbar, dass durch die Verwendung von kationischen Phthalimiden in kosmetischen Blondiermitteln auch der Aufhelleffekt von Haaren verstärkt werden kann.

Aus EP 1 161 936 und DE 10 2005 003 412 sind Verfahren zum Blondieren von menschlichen Haaren auf Peroxydbasis unter Zuhilfenahme von Phthalimidoperoxyhexansäure bzw. Phthalsäureestern vorbekannt.

In nicht vorhersehbarer Weise konnte nun gefunden werden, dass der Einsatz einer Kombination von kationischen Phthalimidderivaten der allgemeinen Formel (I) und Wasserstoffperoxid die Haare viel stärker aufhellt, als es durch den Einsatz einer vergleichbaren Menge Wasserstoffperoxid allein möglich gewesen wäre.

Bedingt durch die verbesserte Blondierleistung bei Anwendung des erfindungsgemäßen Mittels kann die Menge an eingesetztem Oxidationsmittel vermindert und die Haarschädigung hierdurch minimiert werden. Auch eine Verkürzung der Einwirkzeit unter Erzielung eines dem Stand der Technik entsprechenden Aufhelleffekts ist auf diese Weise möglich. Weiterhin verfügen diese Mittel über eine gesteigerte Aufhellleistung gegenüber handelsüblichen Aufhellmitteln und erlauben daher auch die Aufhellung von sehr dunklen Haaren zu hellen Blondnuancen.

Die erfindungsgemäßen Mittel entfärben den natürlichen Farbstoff Melanin durch Oxidation. Auch zuvor auf beziehungsweise in der keratinhaltigen Faser befindliche synthetische Farbstoffe können mit Hilfe der erfindungsgemäßen Mittel zerstört und damit die Faser ausgeblichen werden.

Ein erster Gegenstand der Erfindung ist daher ein Mittel zum Aufhellen von keratinischen Fasern, dadurch

gekennzeichnet, dass es in einem kosmetischen Träger

(i) mindestens ein Oxidationsmittel, ausgewählt aus Wasserstoffperoxid und/oder einem seiner festen Anlagerungsprodukte an organische oder anorganische Verbindungen, und
(ii) mindestens ein kationisches Phthalimid der Formel (I),

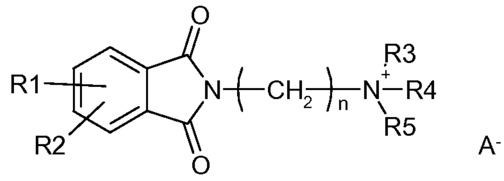

(I),

enthält, worin

| | |
|---|---|
| R1 und R2 | jeweils unabhängig voneinander für ein Wasserstoffatom, ein Halogenatom, eine Hydroxygruppe, eine $C_1$-$C_6$-Alkoxygruppe, stehen, |
| oder R1 und R2 | zusammen mit dem angrenzenden Benzolring eine weitere Phthalimid-Einheit der Formel (Ia) bilden, |

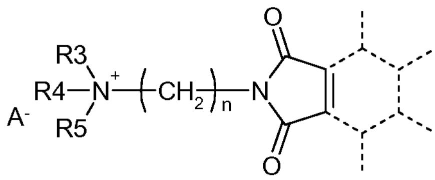

(Ia),

| | |
|---|---|
| R3, R4, R5 | jeweils unabhängig voneinander für eine $C_1$-$C_6$-Alkylgruppe, eine partiell oder vollständig halogenierte $C_1$-$C_6$-Alkylgruppe, eine $C_2$-$C_6$-Alkenylgruppe, eine $C_2$-$C_6$-Hydroxyalkylgruppe, eine $C_2$-$C_6$-Polyhydroxyalkylgruppe, eine $C_1$-$C_6$-Alkoxy-$C_2$-$C_6$-alkylgruppe, eine Cyano-$C_1$-$C_6$-alkylgruppe, eine Aryl- $C_1$-$C_6$-alkylgruppe, eine Carboxy-$C_1$-$C_6$-alkylgruppe oder eine Sulfonyl-$C_1$-$C_6$-alkylgruppe stehen, |
| oder | zwei der Substituenten R3, R4 und R5 zusammen mit dem angebundenen Stickstoff einen 5-, 6- oder 7-gliedrigen, gesättigten oder ungesättigten Ring bilden, der gegebenenfalls weitere Heteroatome enthält, |
| n | für eine ganze Zahl von 1 bis 6 steht und |
| $A^-$ | für ein physiologisch verträgliches Anion steht. |

Unter keratinischen Fasern oder auch Keratinfasern sind dabei Pelze, Wolle, Federn und insbesondere menschliche Haare zu verstehen.

Die erfindungsgemäßen Mittel enthalten die Wirkstoffe in einem kosmetischen Träger. Dieser kosmetische Träger ist bevorzugt wässrig, alkoholisch oder wässrig-alkoholisch. Zum Zwecke der Haarbleiche sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind. Es ist aber auch möglich, zur Lagerung eine pulverförmige oder auch tablettenförmige Formulierung bereitzustellen, was für Aufhellmittel bevorzugt ist. Diese wird dann vor der Anwendung in einem wässrigen Lösungsmittel oder mit organischen Lösungsmitteln bzw. mit Gemischen aus Wasser und organischen Lösungsmitteln unter Erhalt der Anwendungsmischung vermengt. Ein wässriger Träger enthält im Sinne der Erfindung mindestens 40 Gew.-%, insbesondere mindestens 50 Gew.-% Wasser. Unter wässrigalkoholischen Trägern sind im Sinne der vorliegenden Erfindung wasserhaltige Zusammensetzungen, enthaltend 3 bis 70 Gew.-% eines $C_1$-$C_4$-Alkohols, insbesondere Ethanol bzw. Isopropanol, zu verstehen. Die erfindungsgemäßen Mittel können zusätzlich weitere organische Lösungsmittel, wie beispielsweise 4-Methoxybutanol, Ethyldiglykol, 1,2-Propylenglykol, n-Propanol, n-Butanol, n-Butylenglykol, Glycerin, Diethylenglykolmonoethylether, und Diethylenglykolmono-n-butylether, enthalten. Bevorzugt sind dabei alle wasserlöslichen organischen Lösungsmittel. Bevorzugte erfindungsgemäße Mittel sind dadurch gekennzeichnet, dass sie zusätzlich ein nichtwässriges Lösungsmittel enthalten, wobei bevorzugte erfindungsgemäße Mittel das Lösungsmittel in einer Konzentration von 0,1 bis 30 Gew.-%, bevorzugt in einer Konzentration von 1 bis 20 Gew.-%, ganz besonders bevorzugt in einer

Konzentration von 2 bis 10 Gew.-%, jeweils bezogen auf das Mittel, enthalten.

Als erster wesentlicher Inhaltsstoff ist Wasserstoffperoxid und/oder eines seiner festen Anlagerungsprodukte an organische oder anorganische Verbindungen in dem erfindungsgemäßen Mittel enthalten. In einer bevorzugten Ausführungsform wird Wasserstoffperoxid selbst als wässrige Lösung verwendet. Wasserstoffperoxid kann aber auch in Form einer festen Anlagerungsverbindung von Wasserstoffperoxid an anorganische oder organische Verbindungen, wie beispielsweise Natriumperborat, Natriumpercarbonat, Magnesiumpercarbonat, Natriumpercarbamid, Polyvinylpyrrolidinon n $H_2O_2$ (n ist eine positive ganze Zahl größer 0), Harnstoffperoxid und Melaminperoxid, eingesetzt werden. Im letztgenannten Fall setzen die Anlagerungsverbindungen in der erfindungsgemäßen Anwendungsmischung Wasserstoffperoxid frei, d. h. diese Mittel enthalten neben der Anlagerungsverbindung im kosmetischen Träger freies Wasserstoffperoxid.

Erfindungsgemäß bevorzugt wird Wasserstoffperoxid dem erfindungsgemäßen Mittel als wässrige Wasserstoffperoxid-Lösung zudosiert. Eine Ausführungsform des ersten Erfindungsgegenstands ist daher dadurch gekennzeichnet, dass das Mittel als Oxidationsmittel Wasserstoffperoxid als wässrige Lösung enthält.

Die Konzentration einer Wasserstoffperoxid-Lösung wird einerseits von den gesetzlichen Vorgaben und andererseits von dem gewünschten Effekt bestimmt; vorzugsweise werden 6 bis 12 gew.-%ige Lösungen in Wasser verwendet. Erfindungsgemäß bevorzugte Mittel sind dadurch gekennzeichnet, dass sie, bezogen auf ihr Gesamtgewicht, 0,01 bis 12 Gew.-%, vorzugsweise 0,1 bis 10 Gew.-%, besonders bevorzugt 1 bis 6 Gew.-% Wasserstoffperoxid (berechnet als 100%iges $H_2O_2$) enthalten.

Als weiteren, erfindungswesentlichen Inhaltsstoff enthalten die Mittel mindestens einen kationisches Phthalimid der Formel (I). Im Folgenden werden Beispiele für die als Substituenten der Verbindungen der Formel (I) genannten Reste aufgezählt: Beispiele für Halogenatome sind Fluor, Chlor, Brom und Iod;

Beispiele für $C_1$-$C_6$-Alkoxygruppen sind die Gruppen $-OCH_3$, $-OCH_2CH_3$, $-OCH(CH_3)_2$, $-OC(CH_3)_3$, bevorzugt $-OCH_3$;

Eine Ausführungsform der vorliegenden Erfindung ist dadurch gekennzeichnet, dass das Mittel als Phthalimid gemäß Formel (I) mindestens eine Verbindung enthält, bei der die Substituenten R1 und R2 jeweils für ein Wasserstoffatom stehen.

Eine weitere Ausführungsform der vorliegenden Erfindung ist dadurch gekennzeichnet, dass das Mittel als Phthalimid gemäß Formel (I) mindestens eine Verbindung enthält, bei die Substituenten R3, R4 und R5 jeweils unabhängig voneinander für eine $C_1$-$C_6$-Alkylgruppe, eine $C_2$-$C_6$-Alkenylgruppe oder eine $C_2$-$C_6$-Hydroxyalkylgruppe stehen.

Bevorzugte Verbindungen der Formel (I) sind ausgewählt aus Verbindungen 1 bis 28 (Tabelle 1)

| | | | |
|---|---|---|---|
| 1 | 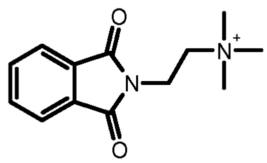 Salze des 2-(1,3-Dioxo-1,3-dihydro-2-isoindol-2-yl)-N,N,N-trimethyl-ethanaminiums | 2 | 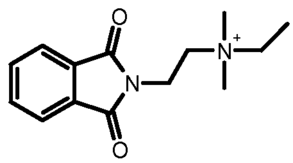 Salze des 2-(1,3-Dioxo-1,3-dihydro-2H-isoindol-2-yl)-N-ethyl-N,N-dimethylethanaminiums |
| 3 | 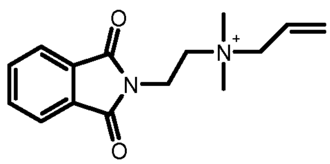 Salze des N-[2-(1,3-Dioxo-1,3-dihydro-2H-isoindol-2-yl)ethyl]-N,N-dimethylprop-2-en-1-aminiums | 4 | 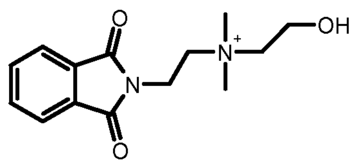 Salze des 2-(1,3-Dioxo-1,3-dihydro-2H-isoindol-2-yl)-N-(2-hyd roxyethyl)-N,N-dimethylethanaminiums |
| 5 | 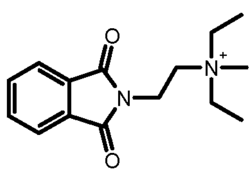 Salze des 2-(1,3-Dioxo-1,3-dihydro-2-isoindol-2-yl)-N,N-diethyl-N-methyl-ethanaminiums | 6 | 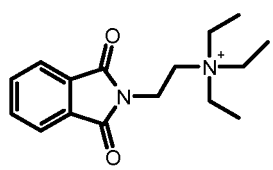 Salze des 2-(1,3-Dioxo-1,3-dihydro-2H-isoindol-2-yl)-N,N,N-triethyl-ethanaminiums |

(fortgesetzt)

| | | | |
|---|---|---|---|
| 7 | 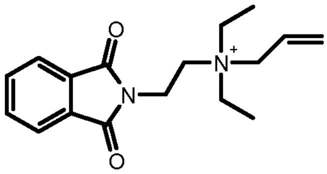<br>Salze des N-[2-(1,3-Dioxo-1,3-dihydro-2H-isoindol-2-yl)ethyl]-N,N-diethylprop-2-en-1-aminiums | 8 | 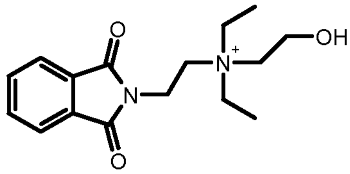<br>Salze des 2-(1,3-Dioxo-1,3-dihydro-2H-isoindol-2-yl)-N-(2-hyd roxyethyl)-N,N-diethylethanaminiums |
| 9 | 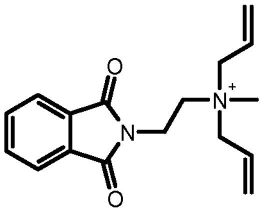<br>Salze des N-Allyl-N-[2-(1,3-dioxo-1,3-dihydro-2H-isoindol-2-yl)ethyl]-N-methyl-prop-2-en-1-aminiums | 10 | 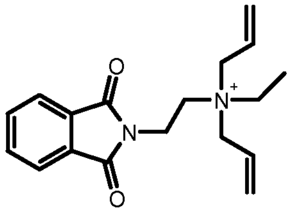<br>Salze des N-Allyl-N-[2-(1,3-dioxo-1,3-dihydro-2H-isoindol-2-yl)ethyl]-N-ethyl-prop-2-en-1-aminiums |
| 11 | 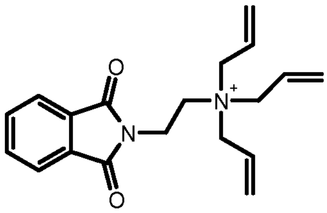<br>Salze des N,N-Diallyl-N-[2-(1,3-dioxo-1,3-dihydro-2H-isoindol-2-yl)ethyl]prop-2-en-1-aminiums | 12 | 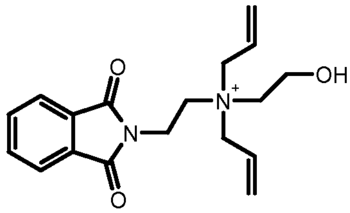<br>Salze des N,N-Diallyl-N-[2-(1,3-dioxo-1,3-dihydro-2H-isoindol-2-yl)ethyl]prop-2-en-1-aminiums |
| 13 | 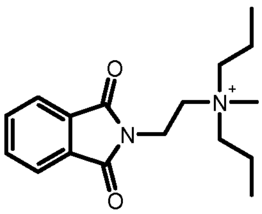<br>Salze des N-[2-(1,3-Dioxo-1,3-dihydro-2H-isoindol-2-yl)ethyl]-N-methyl-N-propylpropan-1-aminiums | 14 | 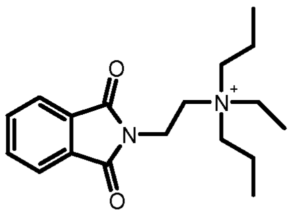<br>Salze des N-[2-(1,3-Dioxo-1,3-dihydro-2H-isoindol-2-yl)ethyl]-N-ethyl-N-propylpropan-1-aminiums |
| 15 | 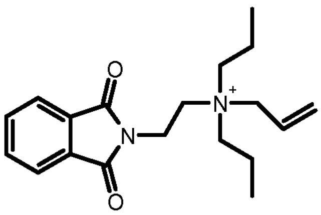<br>Salze des N-[2-(1,3-Dioxo-1,3-dihydro-2H-isoindol-2-yl)ethyl]-N,N-dipropylprop-2-en-1-aminiums | 16 | 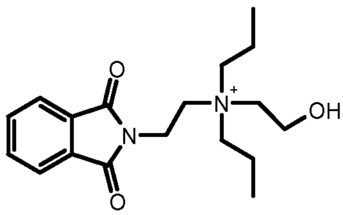<br>Salze des N-[2-(1,3-Dioxo-1,3-dihydro-2H-isoindol-2-yl)ethyl]-N-(2-hydroxy-ethyl)-N-propylpropan-1-aminiums |

(fortgesetzt)

| | | | |
|---|---|---|---|
| 17 | 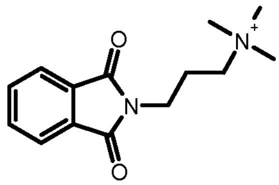<br>Salze des 3-(1,3-Dioxo-1,3-dihydro-2H-isoindol-2-yl)-N,N,N-trimethyl-propan-1-aminiums | 18 | 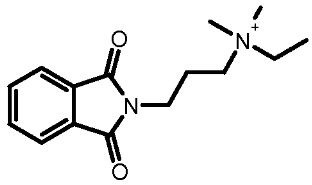<br>Salze des 3-(1,3-Dioxo-1,3-dihydro-2H-isoindol-2-yl)-N-ethyl-N,N-dimethyl-propan-1-aminiums |
| 19 | 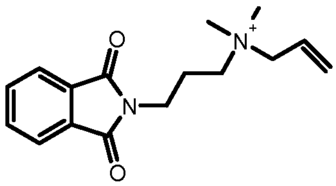<br>Salze des N-[3-(1,3-Dioxo-1,3-dihydro-2H-isoindol-2-yl)propyl]-N,N-dimethyl-prop-2-en-1-aminiums | 20 | 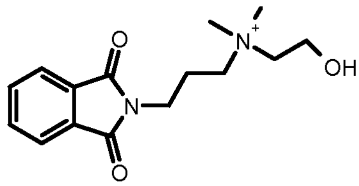<br>Salze des 3-(1,3-Dioxo-1,3-dihydro-2H-isoindol-2-yl)-N-(2-hydroxyethyl)-N,N-dimethylpropan-1-aminiums |
| 21 | 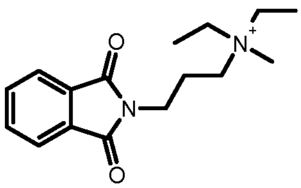<br>Salze des 3-(1,3-Dioxo-1,3-dihydro-2H-isoindol-2-yl)-N,N-diethyl-N-methyl-propan-1-aminiums | 22 | 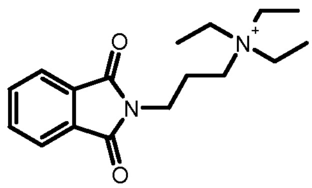<br>Salze des 3-(1,3-Dioxo-1,3-dihydro-2H-isoindol-2-yl)-N,N,N-triethyl-propan-1-aminiums |
| 23 | 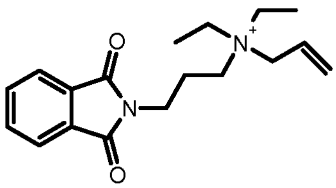<br>N-[3-(1,3-Dioxo-1,3-dihydro-2H-isoindol-2-yl)propyl]-N,N-diethylprop-2-en-1-aminiums | 24 | 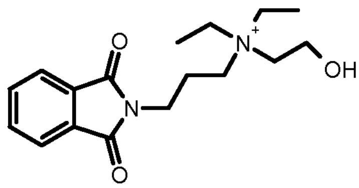<br>Salze des 3-(1,3-Dioxo-1,3-dihydro-2H-isoindol-2-yl)-N,N-diethyl-N-(2-hydroxy-ethyl)-propan-1-aminiums |
| 25 | 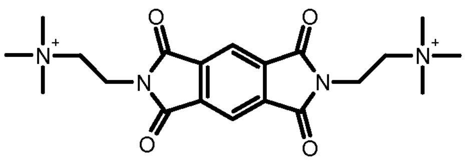<br>Salze des N,N,N-Trimethyl-{1,3,5,7-tetraoxo-6-[2-(trimethylammonio)ethyl]-3,5,6,7-tetrahydropyrrolo[3,4-f]isoindol-2(1H)-yl}ethan-2-aminiums | 26 | 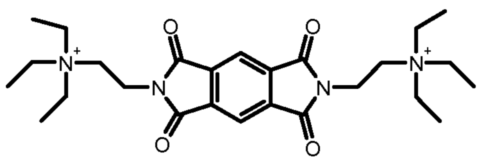<br>Salze des N,N,N-Triethyl-{1,3,5,7-tetraoxo-6-[2-(triethylammonio)ethyl]-3,5,6,7-tetrahydropyrrolo[3,4-f]isoindol-2(1H)-yl}ethan-2-aminiums |
| 27 | 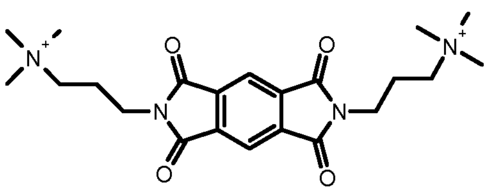 | 28 | 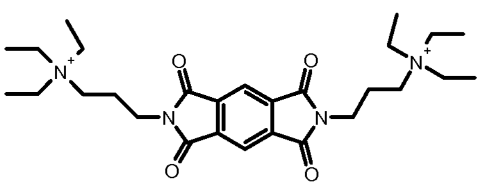 |

(fortgesetzt)

| | | | |
|---|---|---|---|
| | Salze des N,N,N-Trimethyl-{1,3,5,7-tetraoxo-6-[3-(trimethylammonio)-propyl]-3,5,6,7-tetrahydropyrrolo-[3,4-f]isoindol-2(1H)-yl}propan-2-aminiums | | Salze des N,N,N-Triethyl-{1,3,5,7-tetraoxo-6-[3-(triethylammonio)-propyl]-3,5,6,7-tetrahydropyrrolo-[3,4-f]isoindol-2(1H)-yl}propan-2-aminiums |

Dabei sind Mittel bevorzugt, welche in einem kosmetischen Träger neben mindestens einem Oxidationsmittel, ausgewählt aus Wasserstoffperoxid und/oder einem seiner festen Anlagerungsprodukte an organische oder anorganische Verbindungen, mindestens eine Verbindung enthalten, die ausgewählt ist aus der Gruppe von physiologisch verträglichen Salzen, deren kationische Anteile gebildet werden von Salzen des 2-(1,3-Dioxo-1,3-dihydro-2-isoindol-2-yl)-N,N,N-trimethyl-ethanaminiums, Salzen des 2-(1,3-Dioxo-1,3-dihydro-2H-isoindol-2-yl)-N,N-diethyl-N-methylethanaminiums, Salzen des N-[2-(1,3-Dioxo-1,3-dihydro-2H-isoindol-2-yl)ethyl]-N,N-diethylprop-2-en-1-aminiums, Salzen des N-Allyl-N-[2-(1,3-dioxo-1,3-dihydro-2H-isoindol-2-yl)ethyl]-N-methylprop-2-en-1-aminiums, Salzen des N-[2-(1,3-Dioxo-1,3-dihydro-2H-isoindol-2-yl)ethyl]-N-methyl-N-propyl-propan-1-aminiums, Salzen des N-[2-(1,3-Dioxo-1,3-dihydro-2H-isoindol-2-yl)ethyl]-N,N-dipropyl-prop-2-en-1-aminiums, Salzen des 3-(1,3-Dioxo-1,3-dihydro-2H-isoindol-2-yl)-N,N,N-trimethyl-pro-pan-1-aminiums, Salzen des N-[3-(1,3-Dioxo-1,3-dihydro-2H-isoindol-2-yl)propyl]-N,N-dimethyl-prop-2-en-1-aminiums, Salzen des 3-(1,3-Dioxo-1,3-dihydro-2H-isoindol-2-yl)-N,N-diethyl-N-methylpropan-1-aminiums und Salzen des N-[3-(1,3-Dioxo-1,3-dihydro-2H-isoindol-2-yl)propyl]-N,N-diethylprop-2-en-1-aminiums.

Eine bevorzugte Ausführungsform des ersten Erfindungsgegenstands ist dadurch gekennzeichnet, dass das Mittel als Phthalimid gemäß Formel (I) mindestens 2-(1,3-Dioxo-1,3-dihydro-2H-isoindol-2-yl)-N,N-diethyl-N-methylethanaminium-p-toluol-sulfonat, N-Allyl-N-[2-(1,3-dioxo-1,3-dihydro-2H-isoindol-2-yl)ethyl]-N-methylprop-2-en-1-aminium-p-toluolsulfonat und N-[2-(1,3-Dioxo-1,3-dihydro-2H-isoindol-2-yl)ethyl]-N-methyl-N-propylpropan-1-aminium-p-toluolsulfonat enthält.

Erfindungsgemäße Mittel sind bevorzugt dadurch gekennzeichnet, dass das oder die Phthalimid(e) der Formel (I) in einer Menge von 0,01 bis 25 Gew.-%, insbesondere von 0,1 bis 10 Gew.-% und besonders bevorzugt von 0,5 bis 5,0 Gew.-%, jeweils bezogen das Gesamtgewicht des anwendungsbereiten Mittels, enthalten ist.

Die erfindungsgemäßen Mittel können auch direkt vor der Anwendung aus zwei oder mehreren getrennt verpackten Zubereitungen hergestellt werden. Dies bietet sich insbesondere zur Trennung inkompatibler Inhaltsstoffe an, um eine vorzeitige Reaktion zu vermeiden. Ein üblicher Weg besteht daher darin, ein erstes Mittel, welches mindestens ein Phthalimid der allgemeinen Formel (I) enthält, direkt vor der Anwendung mit einem zweiten Mittel, in welchem das oder die erfindungsgemäßen Oxidationsmittel enthalten sind, zu vermischen.

Ein weiterer Gegenstand der vorliegenden Erfindung ist daher ein Mittel zum Aufhellen keratinischer Fasern, insbesondere menschlicher Haare, welches unmittelbar vor dem Aufbringen auf das Haar aus einer fließfähigen Zubereitung (A), die ein Kationisches Phthalimid der allgemeinen Formel (I) enthält, und einer Oxidationsmittelzubereitung (B), enthaltend mindestens ein Oxidationsmittel, ausgewählt aus Wasserstoffperoxid und/oder dessen Anlagerungsverbindungen an organische oder anorganische Verbindungen, erhalten wird.

Die Oxidationsmittelzubereitung (B) ist vorzugsweise eine wässrige, fließfähige Oxidationsmittelzubereitung. Dabei sind bevorzugte erfindungsgemäße Mittel zum Aufhellen keratinischer Fasern dadurch gekennzeichnet, dass die fließfähige Oxidationsmittelzubereitung (B) - bezogen auf ihr Gewicht - 40 bis 90 Gew.-%, vorzugsweise 50 bis 85 Gew.-%, besonders bevorzugt 55 bis 80 Gew.-%, weiter bevorzugt 60 bis 77,5 Gew.-% und insbesondere 65 bis 75 Gew.-% Wasser enthält.

Durch die Verwendung des Kationischen Phthalimids nach Formel (I) wird die Aufhellleistung der erfindungsgemäßen Mittel deutlich gesteigert, so dass unter Umständen auf den Zusatz von weiteren Bleichkraftverstärkern verzichtet werden kann, was zu einer reduzierten Haarschädigung führt.

Für besonders starke Aufhellungen, insbesondere bei sehr dunklen, stark pigmentierten Ausgangshaarfarben, kann es jedoch erforderlich sein, zusätzliche Bleichkraftverstärker in das Mittel einzuarbeiten. Wird eine solche starke Aufhellung gewünscht, so ist es erfindungsgemäß bevorzugt, wenn zusätzlich eine Blondierzubereitung (C), enthaltend mindestens einen Bleichkraftverstärker, der Mischung aus Oxidationsmittelzubereitung (B) und der Zubereitung (A) beigemischt wird. Es kann dabei unerheblich sein, ob zunächst eine Mischung (A) und (B) hergestellt wird, und anschließend die Blondierzubereitung (C) zugemischt wird, oder ob eine davon verschiedene Reihenfolge der Vermischung der einzelnen Komponenten genutzt wird. Es ist bevorzugt, die einzelnen Zubereitungen in möglichst naher zeitlicher Abfolge zu vermischen und das anwendungsbereite Mittel zeitnah auf die keratinischen Fasern zu applizieren.

Eine weitere Ausführungsform der vorliegenden Anmeldung ist daher ein Mittel zum Bleichen keratinischer Fasern, dadurch gekennzeichnet, dass es durch Vermischen von mindestens einer Oxidationsmittelszubereitung (B), enthaltend mindestens ein Oxidationsmittel, ausgewählt aus Wasserstoffperoxid und dessen Anlagerungsverbindungen an feste Träger, mindestens einer Blondierzubereitung (C), enthaltend mindestens einen Bleichkraftverstärker, und mindestens einer Zubereitung (A) hergestellt wird, wobei die Zubereitung (A) in einem kosmetischen Träger mindestens

ein kationisches Phthalimid gemäß Formel (I) enthält.

Als zusätzliche Bleichkraftverstärker der Blondierzubereitung (C) können im Rahmen dieser Erfindung Peroxoverbindungen, weiterhin Verbindungen, die unter Perhydrolysebedingungen aliphatische Peroxocarbonsäuren und/oder substituierte Perbenzoesäure ergeben, Kohlensäurederivate, Alkylcarbonate, -carbamate, Silylcarbonate und -carbamate eingesetzt werden.

Vorzugsweise ist der Bleichkraftverstärker ausgewählt aus Ammoniumperoxodisulfat, Alkalimetallperoxodisulfaten, Ammoniumperoxomonosulfat, Alkalimetallhydrogenperoxomonosulfaten, Alkalimetallperoxodiphosphaten und Erdalkalimetallperoxiden. Besonders bevorzugte Bleichkraftverstärker sind Ammoniumperoxodisulfat, Kaliumperoxodisulfat, Natriumperoxodisulfat, Kaliumhydrogenperoxomonosulfat, Kaliumperoxodiphosphat, Magnesiumperoxid und Bariumperoxid.

Erfindungsgemäß besonders bevorzugt sind Mittel, die in der Blondierzubereitung (C) als Bleichkraftverstärker mindestens ein anorganisches Salz, ausgewählt aus Peroxomonosulfaten und/oder Peroxodisulfaten, enthalten. Weiterhin hat es sich bei den Arbeiten zur vorliegenden Erfindung als besonders bevorzugt erwiesen, wenn die erfindungsgemäßen Mittel mindestens zwei verschiedene Peroxodisulfate enthalten. Bevorzugte Peroxodisulfatsalze sind dabei Kombinationen aus Ammoniumperoxodisulfat und Kaliumperoxodisulfat und/oder Natriumperoxodisulfat. Eine weitere Ausführungsform dieses Erfindungsgegenstands ist daher dadurch gekennzeichnet, dass das Aufhellmittel zusätzlich mindestens ein anorganisches Persulfat- oder Peroxodisulfat-Salz, insbesondere Ammoniumperoxodisulfat, Kaliumperoxodisulfat und/oder Natriumperoxodisulfat, enthält. Die Peroxoverbindungen sind in einer Menge von 0,1 bis 25 Gew.-%, insbesondere in einer Menge von 0,5 bis 15 Gew.-%, bezogen auf das Gesamtgewicht des anwendungsbereiten Mittels, enthalten. Der Einsatz von Persulfatsalzen bzw. Peroxodisulfatsalzen erfolgt in der Regel in Form eines gegebenenfalls entstaubten Pulvers, einer Paste oder eines in Form gepressten Formkörpers.

Die erfindungsgemäßen wasserfreien Zusammensetzungen können anstelle und/oder zusätzlich zu den festen Peroxoverbindungen einen weiteren Bleichkraftverstärker enthalten.

Als Bleichverstärker können Verbindungen, die unter Perhydrolysebedingungen aliphatische Peroxocarbonsäuren mit vorzugsweise 1 bis 10 C-Atomen, insbesondere 2 bis 4 C-Atomen, und/oder gegebenenfalls substituierte Perbenzoesäure ergeben, eingesetzt werden. Geeignet sind Substanzen, die O- und/oder N-Acylgruppen der genannten C-Atomzahl und/oder gegebenenfalls substituierte Benzoylgruppen tragen. Bevorzugt sind mehrfach acylierte Alkylendiamine, insbesondere Tetraacetylethylendiamin (TAED), acylierte Triazinderivate, insbesondere 1,5-Diacetyl-2,4-dioxohexahydro-1,3,5-triazin (DADHT), acylierte Glykolurile, insbesondere Tetraacetylglykoluril (TAGU), N-Acylimide, insbesondere N-Nonanoylsuccinimid (NOSI), acylierte Phenolsulfonate, insbesondere n-Nonanoyl- oder Isononanoyloxybenzolsulfonat (n- bzw. i-NOBS), Carbonsäureanhydride, insbesondere Phthalsäureanhydrid, acylierte mehrwertige Alkohole, insbesondere Triacetin, Ethylenglykoldiacetat und 2,5-Diacetoxy-2,5-dihydrofuran.

Als Bleichverstärker vom Typ der Kohlensäurederivate können erfindungsgemäß bevorzugt Carbonatsalze bzw. Hydrogencarbonatsalze eingesetzt werden. Diese sind bevorzugt ausgewählt aus der Gruppe der Ammonium-, Alkalimetall- (insbesondere Natrium- und Kalium-) sowie Erdalkalimetall- (insbesondere Magnesium- und Calcium-), -carbonatsalze bzw. -hydrogencarbonatsalze. Besonders bevorzugte Carbonat- bzw. Hydrogencarbonatsalze sind Ammoniumhydrogencarbonat, Ammoniumcarbonat, Natriumhydrogencarbonat, Natriumcarbonat, Kaliumhydrogencarbonat, Kaliumcarbonat, Magnesiumcarbonat und Calciumcarbonat. Diese bevorzugten Salze können allein oder in deren Mischungen von mindestens zwei Vertretern als Bleichverstärker verwendet werden.

Als weitere zusätzliche Bleichverstärker können in den erfindungsgemäßen Zusammensetzungen bevorzugt mindestens eine Verbindung ausgewählt aus Essigsäure, Milchsäure, Weinsäure, Zitronensäure, Salicylsäure und ortho-Phthalsäure, enthalten sein.

Die neben oder anstelle von Peroxoverbindungen eingesetzten Bleichkraftverstärker sind in den erfindungsgemäßen, kosmetischen Mitteln bevorzugt in Mengen von 0,05 bis 10 Gew.-%, insbesondere in Mengen von 0,2 bis 5 Gew.-%, jeweils bezogen auf das Gesamtgewicht des anwendungsbereiten Mittels, enthalten.

Obwohl prinzipiell keine Einschränkungen hinsichtlich der Formulierung der Blondierzubereitung (C) bestehen, hat es sich erfindungsgemäß als bevorzugt erwiesen, wenn die Zubereitung (C) wasserfrei formuliert ist.

Wasserfrei im Sinne der vorliegenden Erfindung bedeutet einen Wassergehalt bezogen auf die Zubereitung (C) von weniger als 5 Gew.-%, insbesondere von weniger als 2 Gew.-%. Blondierzubereitungen, die weniger als 0,1 Gew.-% Wasser, enthalten können erfindungsgemäß ganz besonders bevorzugt sein. Die Zubereitung (C) ist vorzugsweise wasserfrei als Pulver oder als Paste formuliert.

Im Falle einer wasserfreien Formulierung hat es sich als besonders bevorzugt erwiesen, wenn die Zubereitung (C) mindestens einen nicht hydroxylierten Fettsäureester mit einem Schmelzpunkt von höchstens 50°C, insbesondere von höchstens 30°C, und/oder mindestens eine $C_{10}$-$C_{30}$-Fettsäure mit mindestens einer zusätzlichen Hydroxygruppe und/oder ein Derivat davon, enthält. Ester von nicht hydroxylierten $C_6$-$C_{30}$-Alkylmonocarbonäuren mit $C_2$-$C_{30}$-Monoalkoholen eignen sich erfindungsgemäß bevorzugt als Fettsäureester. Erfindungsgemäß besonders bevorzugt sind Isopropylmyristat, Isononansäure-cetearylester, 2-Ethylhexylpalmitat, Stearinsäure-2-ethylhexylester, Cetyloleat, Kokosfettalkohol-caprinat/-caprylat, n-Butylstearat, Oleylerucat, Isopropylpalmitat, Oleyloleat, Laurinsäurehexylester, Myristylmyristat, Cetearylisononanoat, Ölsäuredecylester.

Blondierprozesse auf Keratinfasern laufen üblicherweise im alkalischen Milieu ab. Um die Keratinfasern und auch die Haut so weit wie möglich zu schonen, ist die Einstellung eines zu hohen pH-Wertes jedoch nicht wünschenswert. Daher ist es bevorzugt, wenn der pH-Wert des verwendungsbereiten Mittels zwischen 7 und 11, insbesondere zwischen 8 und 10,5, liegt. Bei den pH-Werten im Sinne der vorliegenden Erfindung handelt es sich um pH-Werte, die bei einer Temperatur von 22 °C gemessen wurden.

Die zur Einstellung des bevorzugten pH-Wertes erfindungsgemäß verwendbaren Alkalisierungsmittel können aus der Gruppe, die gebildet wird aus Ammoniak, Alkanolaminen, basischen Aminosäuren, sowie anorganischen Alkalisierungsmitteln wie (Erd-)Alkalimetallhydroxiden, (Erd-)Alkalimetallmetasilikaten, (Erd-)Alkalimetallphosphaten und (Erd-)Alkalimetallhydrogenphosphaten, ausgewählt werden. Als Metallionen dienen bevorzugt Lithium, Natrium und/oder Kalium. Erfindungsgemäß einsetzbare, organische Alkalisierungsmittel werden bevorzugt ausgewählt aus Alkanolaminen aus primären, sekundären oder tertiären Aminen mit einem $C_2$-$C_6$-Alkylgrundkörper, der mindestens eine Hydroxylgruppe trägt. Bevorzugte Alkanolamine sind Monoethanolamin, 2-Amino2-methylpropanol und Triethanolamin. Die als erfindungsgemäßes Alkalisierungsmittel einsetzbaren basischen Aminosäuren werden bevorzugt ausgewählt aus der Gruppe, die gebildet wird aus Arginin, Lysin, Ornithin und Histidin, besonders bevorzugt Arginin. Es hat sich aber im Rahmen der Untersuchungen zur vorliegenden Erfindung herausgestellt, dass weiterhin erfindungsgemäß bevorzugte Mittel dadurch gekennzeichnet sind, dass sie zusätzlich ein organisches Alkalisierungsmittel enthalten. Eine Ausführungsform des ersten Erfindungsgegenstands ist dadurch gekennzeichnet, dass das Mittel zusätzlich mindestens ein Alkalisierungsmittel enthält, welches ausgewählt ist aus der Gruppe, die gebildet wird aus Ammoniak, Alkanolaminen und basischen Aminosäuren, insbesondere aus Ammoniak, Monoethanolamin und Arginin oder seinen verträglichen Salzen.

Es hat sich im Laufe der Untersuchungen der Anmelderin herausgestellt, dass sich die Blondierleistung der Mittel weiterhin steigern lässt, wenn die Aufhellmittel mindestens ein aromatisches, organisches Lösungsmittel enthalten. Aromatische Lösungsmittel im Sinne der Erfindung sind dabei solche Verbindungen, die eine aromatische Struktureinheit, wie beispielsweise eine Phenylgruppe, in ihrer Strukturformel aufweisen und weiterhin unter Normalbedingungen, also Raumtemperatur und Normaldruck, flüssig sind. Vorzugsweise handelt es sich dabei um carbocyclische Lösungsmittel, die bevorzugt zusätzlich eine Hydroxygruppe tragen. Bevorzugte Beispiele für solche aromatischen Lösungsmittel sind Alkohole, wie Benzylalkohol, 2-Phenylethylalkohol, 1-Phenylethylalkohol, 2-Phenoxyethanol, 3-Methylbenzylalkohol, 2-Methoxybenzylalkohol und 3-Methoxybenzylalkohol.

Ein erfindungsgemäß ganz besonders bevorzugtes aromatisches Lösungsmittel ist Benzylalkohol. Es sind dabei erfindungsgemäße Mittel bevorzugt, die 0,01 bis 15 Gew.-%, insbesondere 0,1 bis 10 Gew.-% und besonders bevorzugt 0,5 bis 5,0 Gew.-%, jeweils bezogen auf das Gesamtgewicht des anwendungsbereiten Mittels, mindestens eines aromatischen, organischen Lösungsmittels enthalten.

Erfindungsgemäß kann das Aufhellmittel auch zusammen mit einem Katalysator auf das Haar aufgebracht werden. Solche Katalysatoren sind z. B. bestimmte Enzyme, insbesondere Peroxidasen, Iodide, Chinone oder Metallionen, wie beispielsweise $Zn^{2+}$, $Cu^{2+}$, $Fe^{2+}$, $Fe^{3+}$, $Mn^{2+}$, $Mn^{4+}$, $Li^{+}$, $Mg^{2+}$, $Ca^{2+}$, $Ce^{4+}$, $V^{3+}$, $Co^{2+}$, $Ru^{3+}$ und $Al^{3+}$, insbesondere $Zn^{2+}$, $Cu^{2+}$ und $Mn^{2+}$.

Weiterhin hat es sich als vorteilhaft erweisen, wenn die Aufhellmittel mindestens einen Stabilisator oder Komplexbildner enthalten. Besonders bevorzugte Stabilisatoren sind Phenacetin, Alkalibenzoate (Natriumbenzoat) und Salicylsäure. Weiterhin können alle Komplexbildner des Standes der Technik eingesetzt werden. Diese können unterschiedlichen chemischen Gruppen angehören. Vorzugsweise werden einzeln oder im Gemisch miteinander eingesetzt. Erfindungsgemäß bevorzugte Komplexbildner sind stickstoffhaltigen Polycarbonsäuren, insbesondere EDTA, und Phosphonate, vorzugsweise Hydroxyalkan- bzw. Aminoalkanphosphonate und insbesondere 1-Hydroxyethan-1,1-diphosphonat (HEDP) bzw. dessen Di- oder Tetranatriumsalz und/oder Ethylendiamintetramethylenphosphonat (EDTMP) bzw. dessen Hexanatriumsalz und/oder Diethylentriaminpentamethylenphosphonat (DTPMP) bzw. dessen Hepta- oder Octanatriumsalz.

Die anwendungsbereiten Aufhellmittel können weiterhin zusätzliche Wirk-, Hilfs- und Zusatzstoffe enthalten.

Vorzugsweise werden die anwendungsbereiten Aufhellmittel als fließfähigen Zubereitung bereitgestellt und ihm daher zusätzlich ein Emulgator bzw. ein Tensid zugesetzt, wobei oberflächenaktive Substanzen je nach Anwendungsgebiet als Tenside oder als Emulgatoren bezeichnet werden und aus anionischen, kationischen, zwitterionischen, amphoteren und nichtionischen Tensiden und Emulgatoren ausgewählt sind.

Erfindungsgemäß bevorzugte Mittel sind dadurch gekennzeichnet, dass das Mittel zusätzlich mindestens ein anionisches Tensid enthält. Bevorzugte anionische Tenside sind Fettsäuren, Alkylsulfate, Alkylethersulfate und Ethercarbonsäuren mit 10 bis 20 C-Atomen in der Alkylgruppe und bis zu 16 Glykolethergruppen im Molekül. Die anionischen Tenside werden in Mengen von 0,1 bis 45 Gew.%, bevorzugt 1 bis 30 Gew.% und ganz besonders bevorzugt von 1 bis 15 Gew.%, bezogen auf die Gesamtmenge des anwendungsbereiten Mittels, eingesetzt.

Erfindungsgemäß bevorzugte Mittel sind dadurch gekennzeichnet, dass das Mittel zusätzlich mindestens ein zwitterionisches Tensid enthält. Besonders geeignete zwitterionische Tenside sind die sogenannten Betaine und N-Alkyl-N,N-dimethylammonium-glycinate, N-Acyl-aminopropyl-N,N-dimethylammoniumglycinate, und 2-Alkyl-3-carboxymethyl-3-hydroxyethyl-imidazoline. Ein bevorzugtes zwitterionisches Tensid ist das unter der INCI-Bezeichnung Cocamidopropyl Betaine bekannte Fettsäureamid-Derivat.

Erfindungsgemäß bevorzugte Mittel sind dadurch gekennzeichnet, dass das Mittel zusätzlich mindestens ein amphoteres Tensid enthält. Beispiele für geeignete amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren. Besonders bevorzugte amphotere Tenside sind das N-Kokosalkylaminopropionat, das Kokosacylaminoethylaminopropionat und das $C_{12}$-$C_{18}$-Acylsarcosin.

Weiterhin hat es sich als vorteilhaft erwiesen, wenn die Mittel weitere, nichtionogene grenzflächenaktive Stoffe, enthalten. Als bevorzugte nichtionische Tenside haben sich Alkylpolyglykoside sowie Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Die nichtionischen, zwitterionischen oder amphoteren Tenside werden in Mengen von 0,1 bis 45 Gew.%, bevorzugt 1 bis 30 Gew.% und ganz besonders bevorzugt von 1 bis 15 Gew.%, bezogen auf die Gesamtmenge der anwendungsbereiten Mittel, eingesetzt.

Erfindungsgemäß geeignete Mittel können auch kationische Tenside vom Typ der quartären Ammoniumverbindungen, der Esterquats und der Amidoamine enthalten. Bevorzugte quaternäre Ammoniumverbindungen sind Ammoniumhalogenide sowie die unter den INCI-Bezeichnungen Quaternium-27 und Quaternium-83 bekannten Imidazolium-Verbindungen. Weitere erfindungsgemäß verwendbare kationische Tenside stellen die quaternisierten Proteinhydrolysate dar. Eine erfindungsgemäß besonders geeignete Verbindung aus der Amidoamine stellt das unter der Bezeichnung Tegoamid® S 18 im Handel erhältliche Stearamidopropyl-dimethylamin dar. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex®, Dehyquart® und Armocare® vertrieben. Die kationischen Tenside sind in den erfindungsgemäß verwendeten Mitteln bevorzugt in Mengen von 0,05 bis 10 Gew.-%, bezogen auf das gesamte Mittel, enthalten. Mengen von 0,1 bis 5 Gew.-% sind besonders bevorzugt.

Die anwendungsbereiten Aufhellmittel können weitere Hilfs- und Zusatzstoffe enthalten. So hat es sich als vorteilhaft erwiesen, wenn die Mittel mindestens ein Verdickungsmittel enthält. Bezüglich dieser Verdickungsmittel bestehen keine prinzipiellen Einschränkungen. Es können sowohl organische als auch rein anorganische Verdickungsmittel zum Einsatz kommen.

Geeignete Verdickungsmittel sind

- anionische, synthetische Polymere;
- kationische, synthetische Polymere;
- natürlich vorkommende Verdickungsmittel, wie nichtionische Guargums, Skleroglucangums oder Xanthangums, Gummi arabicum, Ghatti-Gummi, Karaya-Gummi, Tragant-Gummi, Carrageen-Gummi, Agar-Agar, Johannisbrotkernmehl, Pektine, Alginate, Stärke-Fraktionen und Derivate wie Amylose, Amylopektin und Dextrine, sowie Cellulosederivate, wie beispielsweise Methylcellulose, Carboxyalkylcellulosen und Hydroxyalkylcellulosen;
- nichtionische, vollsynthetische Polymere, wie Polyvinylalkohol oder Polyvinylpyrrolidinon; sowie
- anorganische Verdickungsmittel, insbesondere Schichtsilikate wie beispielsweise Bentonit, besonders Smektite, wie Montmorillonit oder Hectorit.

Zur weiteren Steigerung der Aufhellung können der erfindungsgemäßen Zusammensetzung zusätzlich mindestens eine $SiO_2$-Verbindung wie Kieselsäure oder Silicate, insbesondere Wassergläser, zugesetzt sein. Es kann erfindungsgemäß bevorzugt sein, die $SiO_2$-Verbindunge in Mengen von 0,05 Gew.-% bis 15 Gew.-%, besonders bevorzugt in Mengen von 0,15 Gew.-% bis 10 Gew.-% und ganz besonders bevorzugt in Mengen von 0,2 Gew.-% bis 5 Gew.-%, jeweils bezogen auf die erfindungsgemäße wasserfreie Zusammensetzung, einzusetzen. Die Mengenangaben geben dabei jeweils den Gehalt der $SiO_2$-Verbindunge (ohne deren Wasseranteil) in den Mitteln wieder.

Weiterhin können die Aufhellmittel zur Mattierung von unerwünschten Restfarbeindrücken, insbesondere im rötlichen oder bläulichen Bereich, bestimmte, direktziehende Farbstoffe der komplementären Farben enthalten. Dabei handelt es sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anterachinone oder Indophenole. Direktziehende Farbstoffe sind als anionische, kationische und nichtionische direktziehende Farbstoffe bekannt. Die direktziehenden Farbstoffe werden jeweils bevorzugt in einer Menge von 0,001 bis 2 Gew.-%, bezogen auf die gesamte Anwendungszubereitung, eingesetzt.

Bevorzugte anionische direktziehende Farbstoffe sind die unter den internationalen Bezeichnungen bzw. Handelsnamen Acid Yellow 1, Yellow 10, Acid Yellow 23, Acid Yellow 36, Acid Orange 7, Acid Red 33, Acid Red 52, Pigment

Red 57:1, Acid Blue 7, Acid Green 50, Acid Violet 43, Acid Black 1, Acid Black 52, Bromphenolblau und Tetrabromphenolblau bekannten Verbindungen.

Bevorzugte kationische direktziehende Farbstoffe sind kationische Triphenylmethanfarbstoffe, wie beispielsweise Basic Blue 7, Basic Blue 26, Basic Violet 2 und Basic Violet 14, aromatische Systeme, die mit einer quaternären Stickstoffgruppe substituiert sind, wie beispielsweise Basic Yellow 57, Basic Red 76, Basic Blue 99, Basic Brown 16 und Basic Brown 17, kationische Anthrachinonfarbstoffe, wie HC Blue 16 (Bluequat B) sowie direktziehende Farbstoffe, die einen Heterocyclus enthalten, der mindestens ein quaternäres Stickstoffatom aufweist, insbesondere Basic Yellow 87, Basic Orange 31 und Basic Red 51. Die kationischen direktziehenden Farbstoffe, die unter dem Warenzeichen Arianor vertrieben werden, sind erfindungsgemäß ebenfalls bevorzugte kationische direktziehende Farbstoffe.

Als nichtionische direktziehende Farbstoffe eignen sich insbesondere nichtionische Nitro- und Chinonfarbstoffe und neutrale Azofarbstoffe. Bevorzugte nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)aminophenol, 2-(2-Hydroxyethyl)amino-4,6-dinitro-phenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)amino]-benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Ganz besonders bevorzugt sind Aufhellmittel, die mindestens eine Kombination aus Tetrabromphenolblau und Acid Red 92 enthalten.

Ferner können die erfindungsgemäßen Mittel weitere Wirk-, Hilfs- und Zusatzstoffe enthalten, wie beispielsweise nichtionische Polymere wie beispielsweise Vinylpyrrolidinon/Vinylacrylat-Copolymere, Polyvinylpyrrolidinon, Vinylpyrrolidinon/Vinylacetat-Copolymere, Polyethylenglykole und Polysiloxane; zusätzliche Silikone wie flüchtige oder nicht flüchtige, geradkettige, verzweigte oder cyclische, vernetzte oder nicht vernetzte Polyalkylsiloxane (wie Dimethicone oder Cyclomethicone), Polyarylsiloxane und/oder Polyalkylarylsiloxane, insbesondere Polysiloxane mit organofunktionelle Gruppen, wie substituierten oder unsubstituierten Aminen (Amodimethicone), Carboxyl-, Alkoxy- und/oder Hydroxylgruppen (Dimethiconcopolyole), lineare Polysiloxan(A)-Polyoxyalkylen(B)-Blockcopolymere, gepfropften Silikonpolymere; kationische Polymere wie quaternisierte Celluloseether, Polysiloxane mit quaternären Gruppen, Dimethyldiallylammoniumchlorid-Polymere, Acrylamid-Dimethyldiallyl-ammoniumchlorid-Copolymere, mit Diethylsulfat quaternierte Dimethylamino-ethylmethacrylat-Vinylpyrrolidinon-Copolymere, Vinylpyrrolidinon-Imidazolinium-methochlorid-Copolymere und quaternierter Polyvinylalkohol; zwitterionische und amphotere Polymere; anionische Polymere wie beispielsweise Polyacrylsäuren oder vernetzte Polyacrylsäuren; Strukturanten wie Glucose, Maleinsäure und Milchsäure, haarkonditionierende Verbindungen wie Phospholipide, beispielsweise Lecitin und Kephaline; Parfümöle, Dimethylisosorbid und Cyclodextrine; faserstrukturverbessernde Wirkstoffe, insbesondere Mono-, Di- und Oligosaccharide wie beispielsweise Glucose, Galactose, Fructose, Fruchtzucker und Lactose; Farbstoffe zum Anfärben des Mittels; Antischuppenwirkstoffe wie Piroctone Olamine, Zink Omadine und Climbazol; Aminosäuren und Oligopeptide, insbesondere Arginin und/oder Serin; Proteinhydrolysate auf tierischer und/oder pflanzlicher Basis, wie beispielsweise Elastin-, Kollagen-, Keratin-, Seiden- und Milcheiweiß-Proteinhydrolysate, oder Mandel-, Reis-, Erbsen-, Kartoffel- und Weizenproteinhydrolysate, sowie in Form ihrer Fettsäure-Kondensationsprodukte oder gegebenenfalls anionisch oder kationisch modifizierten Derivate; pflanzliche Öle, wie Macadamianussöl, Palmöl, Amaranthsamenöl, Pfirsichkernöl, Avocadoöl, Olivenöl, Kokosöl, Rapsöl, Sesamöl, Jojobaöl, Sojaöl, Erdnussöl, Nachtkerzenöl und Teebaumöl; Lichtschutzmittel, wie derivatisierte Benzophenone, Zimtsäure-Derivate und Triazine; Wirkstoffe wie Panthenol, Pantothensäure, Pantolacton, Allantoin, Pyrrolidinoncarbonsäuren und deren Salze sowie Bisabolol; Polyphenole, insbesondere Hydroxyzimtsäuren, 6,7-Dihydroxycumarine, Hydroxybenzoesäuren, Catechine, Tannine, Leukoanthocyanidine, Anthocyanidine, Flavanone, Flavone und Flavonole; Ceramide oder Pseudoceramide; Vitamine, Provitamine und Vitaminvorstufen, insbesondere der Gruppen A, $B_3$, $B_5$, $B_6$, C, E, F und H; Pflanzenextrakte wie beispielsweise die Extrakte aus Aloe Vera, Angelica, Anis, Aprikose, Benzoe, Bergamotte, Birke, Brennnessel, Calmus, Cassis, Costus, Eibisch, Eichenrinde, Elemi, Estragon, Fichtennadel, Galbanum, Geranium, Ginseng, Grapefruit, Guajakholz, grünem Tee, Hamamelis, Hauhechel, Hopfen, Huflattich, Ingwerwurzel, Iris, Jasmin, Kamille, Kardamon, Klee, Klettenwurzel, Kiefer, Kiwi, Kokosnuss, Koriander, Kümmel, Latschen, Lavendel, Lemongras, Lilie, Limone, Lindenblüten, Litchi, Macis, Malve, Mandel, Mango, Melisse, Melone, Meristem, Myrrhe, Neroli, Olibanum, Opoponax, Orange, Patchouli, Petitgrain, Pinie, Quendel, Rooibos, Rosen, Rosmarin, Rosskastanie, Sandelholz, Salbei, Schachtelhalm, Schafgarbe, Sellerie, Tanne, Thymian, Wacholder, Weinblättern, Weißdorn, Weizen, Wiesenschaumkraut, Ylang-Ylang, Zeder und Zitrone; Fette und Wachse wie Fettalkohole, Bienenwachs, Montanwachs und Paraffine; Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate; Trübungsmittel wie Latex, Styrol/PVP- und Styrol/Acrylamid-Copolymere; Perlglanzmittel wie Ethylenglykolmono- und -distearat

sowie PEG-3-distearat; Pigmente sowie Treibmittel wie Propan-Butan-Gemische, $N_2O$, Dimethylether, $CO_2$ und Luft.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher verwiesen. Die zusätzlichen Wirk- und Hilfsstoffe werden in den erfindungsgemäßen Mitteln bevorzugt in Mengen von jeweils 0,0001 bis 10 Gew.-%, insbesondere von 0,0005 bis 5 Gew.-%, bezogen auf das Gesamtgewicht der Anwendungsmischung, eingesetzt.

Ein weiterer Gegenstand der vorliegenden Erfindung ist ein Verfahren zum Aufhellen von keratinischen Fasern, insbesondere menschlichen Haaren, welches dadurch gekennzeichnet ist, dass ein Mittel des ersten Erfindungsgegenstands auf die keratinhaltigen Fasern aufgebracht, 5 bis 60 Minuten auf der Faser belassen und anschließend mit Wasser wieder ausgespült oder mit einem Shampoo ausgewaschen wird. Bevorzugt beträgt die Einwirkzeit der anwendungsbereiten Aufhellmittel 5 bis 45 min, insbesondere 10 bis 40 min, besonders bevorzugt 15 bis 35 min. Während der Einwirkzeit des Mittels auf der Faser kann es vorteilhaft sein, den Aufhellvorgang durch Wärmezufuhr zu unterstützen. Die Wärmezufuhr kann durch eine externe Wärmequelle, wie z.B. warme Luft eines Warmluftgebläses, als auch, insbesondere bei einer Haaraufhellung am lebenden Probanden, durch die Körpertemperatur des Probanden erfolgen. Bei letzterer Möglichkeit wird üblicherweise die aufzuhellende Partie mit einer Haube abgedeckt. Eine Einwirkphase bei Raumtemperatur ist ebenfalls erfindungsgemäß. Insbesondere liegt die Temperatur während der Einwirkzeit zwischen 20 °C und 40 °C, insbesondere zwischen 25 °C und 38 °C. Die Aufhellmittel ergeben bereits bei physiologisch verträglichen Temperaturen von unter 45°C gute Blondier- und Aufhellergebnisse. Nach Ende der Einwirkzeit wird die verbleibende Aufhellzubereitung mit Wasser oder einem Reinigungsmittel aus dem Haar gespült. Als Reinigungsmittel kann dabei insbesondere handelsübliches Shampoo dienen, wobei insbesondere dann auf das Reinigungsmittel verzichtet werden kann und der Ausspülvorgang mit Wasser erfolgen kann, wenn das Aufhellmittel einen stark tensidhaltigen Träger besitzt.

Ein weiterer Gegenstand der vorliegenden Erfindung ist die kosmetische Verwendung eines Mittels des ersten Erfindungsgegenstands zum Aufhellen von keratinhalten Fasern, insbesondere menschlichen Haaren.

Die Ausführungsformen des ersten Erfindungsgegenstands gelten mutatis mutandis für das Verfahren des zweiten Erfindungsgegenstands und die Verwendung des dritten Erfindungsgegenstands.

Die Konfektionierung der erfindungsgemäßen Aufhellmittel unterliegt prinzipiell keinerlei Beschränkungen. Üblicherweise werden die erfindungsgemäßen Mittel als 1-Komponentenmittel (A) konfektioniert, die gegebenenfalls unmittelbar vor der Anwendung mit einer zweiten Zubereitung, enthaltend ein Oxidationsmittel, vermischt werden. Es hat sich aber als bevorzugt erwiesen, wenn das Produkt als 2-Komponentenmittel konfektioniert ist. Die beiden Zubereitungen werden vor der Anwendung vermischt.

Um eine vorzeitige, unerwünschte Reaktion der Phthalimide der allgemeinen Formel (I) mit dem Oxidationsmittel zu verhindern, werden die Phthalimide der Formel (I) zweckmäßiger von der Oxidationsmittelzubereitung getrennt konfektioniert und erst unmittelbar vor der Anwendung in Kontakt gebracht.

Ein weiterer Gegenstand der vorliegenden Erfindung ist daher ein Mittel, welches dadurch gekennzeichnet ist, dass es unmittelbar vor der Anwendung durch Vermischen mindestens zweier Zubereitungen hergestellt wird, wobei die mindestens zwei Zubereitungen in mindestens zwei getrennt konfektionierten Containern bereitgestellt werden und wobei ein Container ein Mittel (A), welches in einem kosmetischen Träger mindestens ein kationisches Phthalimid der allgemeinen Formel (I) enthält, und ein weiterer Container eine Oxidationsmittelzubereitung (B), enthaltend mindestens ein Oxidationsmittel, ausgewählt aus Wasserstoffperoxid und/oder dessen Anlagerungsverbindungen an organische oder anorganische Verbindungen, enthält. Um dem Anwender die Komponenten des anwendungsbereiten Aufhellmittels möglichst komfortabel anzubieten, ist es sinnvoll, die einzelnen Zubereitungen in einer Verpackungseinheit gemeinsam zu vertreiben.

Ein weiterer Gegenstand der vorliegenden Erfindung ist daher eine Mehrkomponentenverpackungseinheit (Kit-of-Parts) zum Aufhellen keratinischer Fasern, dadurch gekennzeichnet, dass sie in getrennt voneinander konfektionierten Containern mindestens eine Oxidationsmittelzubereitung (B), enthaltend Wasserstoffperoxid oder eine festen Anlagerungsverbindung von Wasserstoffperoxid an anorganische oder organische Verbindungen, und mindestens eine Zubereitung (A) enthält, wobei die Zubereitung (A) in einem kosmetischen Träger mindestens ein Phthalimid der Formel (I) enthält.

Zur Steigerung der Aufhellleistung kann die Mehrkomponentenverpackungseinheit zusätzlich mindestens eine Blondierzubereitung (C) enthalten. Eine weitere Ausführungsform dieses Erfindungsgegenstands ist daher dadurch gekennzeichnet, dass das Kit-of-Parts zusätzlich in einem getrennt konfektionierten Container mindestens ein Blondierpulver (C), enthaltend mindestens ein anorganisches Peroxodisulfatsalz, ausgewählt aus Ammoniumperoxodisulfat, Kaliumperoxodisulfat und/oder Natriumperoxodisulfat, enthält.

Die Komponenten der Mehrverpackungseinheit werden getrennt voneinander in räumlich unterschiedlichen Behältern oder Containern konfektioniert. Der Begriff "Behälter" kennzeichnet hierbei eine Aufnahmemöglichkeit, unabhängig von deren Form, Material oder Verschluss, welche die Möglichkeit beinhaltet, Stoffe oder Stoffgemische zu beinhalten. Der Begriff Behälter umfasst daher, ohne darauf beschränkt zu sein, das Innere einer Tube, eines Beutels

oder Sackes, eines Kanisters, einer Dose, einer Wanne, einer Flasche, eines Glases oder eines Paketes, eines Kartons, einer Box, eines Umschlages oder eines anderen Behältnisses. Die Behälter können mit einer wiederverschließbaren Öffnung wie einem Schraubverschluss versehen sein. Dies kann insbesondere dann von Vorteil sein, wenn mehrere Mittel vor der Anwendung durch Schütteln innig miteinander vermischt werden sollten.

Die Komponenten der Aufhellzubereitung können in einem Doppelkammer-Behälter mit getrennter oder gemeinsamer Öffnung enthalten sein. Es ist jedoch bevorzugt, sie auf verschiedene Behälter aufzuteilen und den Verbraucher anzuleiten, sie vor der Anwendung miteinander zu mischen. Bevorzugt enthält die Mehrkomponentenverpackungseinheit (Kit-of-Parts) zusätzlich eine Gebrauchsanleitung. Die Gebrauchsanleitung enthält insbesondere Informationen, Erläuterungen und gegebenenfalls Illustrationen für den Verbraucher (m/f) zur Anwendung der Mittel aus den Behältern der Verpackungseinheit in einem Verfahren gemäß dem zweiten Erfindungsgegenstand. Darüber hinaus kann es bevorzugt sein, wenn weiterhin eine Anmischhilfe, wie beispielsweise eine Schale, eine Applikationshilfe, wie beispielsweise ein Kamm oder ein Pinsel, und/oder eine persönliche Schutzausrüstung, wie beispielsweise Einweg-Handschuhe dem Kit beigefügt sind.

Bezüglich bevorzugter Ausführungsformen der erfindungsgemäßen Mehrkomponentenverpackungseinheit gelten mutatis mutandis die Ausführungsformen der vorangegangenen Erfindungsgegenstände.

**Beispiele**

**1. Synthesebeispiele**

**1.1. Synthese von 2-[2-(Diethylamino)ethyl]-1H-isoindol-1,3(2H)-dion**

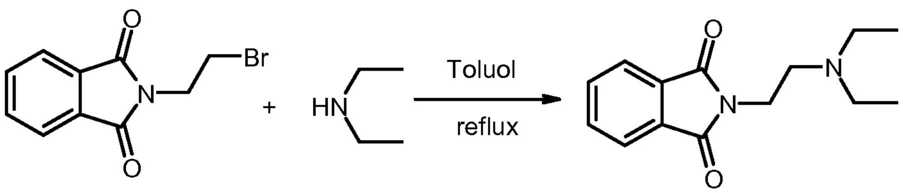


25,4 g (0,10 mol) N-(2-Bromethyl)phthalimid wurden zusammen mit der vierfachen molaren Menge Diethylamin (29,2 g; 0,40 mol) in 500 ml Toluol gelöst und für 8 Stunden unter Rückfluss erhitzt. Nach der Reaktion wurden überschüssiges Diethylamin und das Lösungsmittel im Vakuum am Rotationsverdampfer entfernt. Der Rückstand wurde mit *tert*-Butylmethylether versetzt und durch Filtrieren von den ausgefallenen Salzen befreit. Anschließend wurde die Etherphase wieder komplett eingeengt. Ausbeute: 21,0 g (85,4 %).

**1.2. Synthese von 2-(1,3-Dioxo-1,3-dihydro-2H-isoindol-2-yl)-N,N-diethyl-N-methyl-ethanaminium-p-toluolsulfonat (Aktivator 5)**

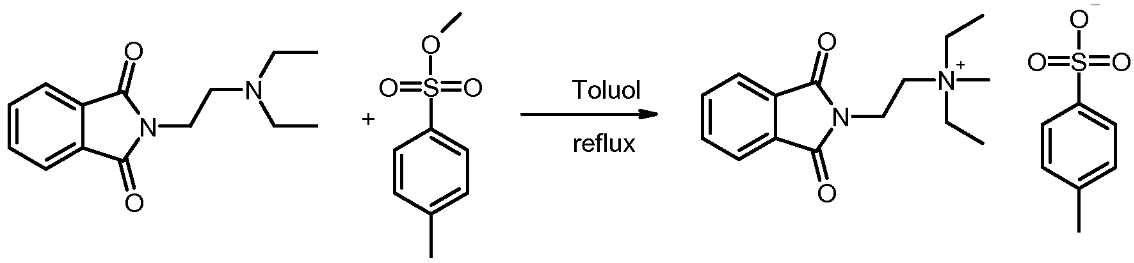


20 ,0 g (81,3 mmol) 2-[2-(Diethylamino)ethyl]-1H-isoindol-1,3(2H)-dion aus Stufe 1 und 16,6 g (89,5 mmol) p-Toluolsulfonsäuremethylester wurden in 250 ml Toluol für 8 Stunden unter Rückfluss erhitzt. Nach dem Abkühlen wurde der weiße, kristalline Feststoff abfiltriert und mit Toluol nachgewaschen. Anschließend wurde das Produkt im Vakuum bei 30 °C getrocknet. Ausbeute: 10,3 g (29,3 %); $^{1}$H-NMR (400 MHz, DMSO-$d_6$): δ [ppm] = 1,39 (t, 6H); 2,28 (s, 3H); 3,06 (s, 3H); 3,47 (m, 6H); 3,97 (q, 2H); 7,10 (d, 2H); 7,48 (d, 2H); 7,89 (m, 4H); $^{13}$C-NMR (400 MHz, DMSO-$d_6$): δ [ppm]

= 8,1; 20,7; 30,5; 46,1; 55,5; 56,0; 123,2; 125,4; 128,0; 131,2; 134,5; 137,6; 145,6; 167,3.

**2. Beispiele zur Blondierung - Blondierungen mit Wasserstoffperoxid**

**2.1. Herstellung einer Blondiercreme**

Aus den aufgelisteten Bestandteilen wurden Blondiercremes wie folgt hergestellt:

| Rohstoff | Gew.-% | |
|---|---|---|
| | V1 | E1 |
| Hydrenol D | 12,00 | 12,00 |
| Lorol. techn. | 2,40 | 2,40 |
| Texapon NSO | 26,50 | 26,50 |
| Stabylen 30 | 0,10 | 0,10 |
| Cetiol OE | 2,40 | 2,40 |
| Turpinal SL | 0,20 | 0,20 |
| Natriumsilikat 40/42 | 0,50 | 0,50 |
| Ammoniumsulfat | 1,00 | 1,00 |
| Ammoniak, 25% | 7,60 | 7,60 |
| **2-(1,3-Dioxo-1,3-dihydro-2H-isoindol-2-yl)-N,N-diethyl-N-methyl-ethanaminium-p-toluolsulfonat (Aktivator 5)** | --- | 2,00 |
| Wasser | ad 100 | ad 100 |

| | |
|---|---|
| Hydrenol® D | INCI-Bezeichnung: Cetearyl alcohol (Cognis) |
| Lorol® tech. | INCI-Bezeichnung: Coconut alcohol (Cognis) |
| Texapono® NSO | ca. 27,5% Aktivsubstanz; INCI-Bezeichnung: Sodium Laureth Sulfate (Cognis) |
| Stabylen® 30 | INCI-Bezeichnung: Acrylates/Vinylisodecanoate Crosspolymer (3V Sigma) |
| Cetiol OE | INCI-Bezeichnung: Dicaprylether (Cognis) |
| Turpinal® SL | ca. 58 - 61% Aktivsubstanzgehalt; INCI-Bezeichnung: Etidronic Acid, Aqua (Solutia) |

Hydrenol D und Lorol wurden zusammen bei 80 °C aufgeschmolzen, dann wurden Texapon NSO, Stabylen 30, Cetiol OE und Turpinal SL der Reihe nach unter Rühren eingearbeitet. Natriumsilikat 40/42 und Ammoniumsulfat wurden jeweils in einer kleinen Menge Wasser vorgelöst und ebenfalls unter Rühren hinzugefügt. Bei einer Temperatur von ca. 40 °C wurde schließlich der Ammoniak hinzugegeben. Unter Rühren wurde mit Wasser auf 100 % aufgefüllt und die Formulierung kalt gerührt.

**2.2. Vermischen mit der Entwicklerdispersion**

Die unter 2.1.hergestellten Blondiercremes wurden jeweils im Verhältnis 1:1 mit einer wie folgt zusammengesetzten Entwicklerdispersion ausgemischt.

| **Rohstoff** | Gew.-% |
|---|---|
| Ammoniak, 25 % | 0,62 |
| Dipicolinsäure | 0,10 |
| Dinatriumpyrophosphat | 0,03 |
| Turpinal SL | 1,50 |
| Texapon NSO | 2,00 |
| Dow Corning DB 110 A (nicht ionische Silikonemulsion) | 0,07 |

(fortgesetzt)

| Rohstoff | Gew.-% |
|---|---|
| Aculyn 33A (Acrylpolymer) | 12,00 |
| Wasserstoffperoxid 50 % | 22,40 |
| Wasser | ad 100 |

Aculyn® 33A ca. 28% Festkörper in Wasser; INCI-Bezeichnung: Acrylates Copolymer

Für den Blondierprozeß wurde auf Strähnen dunkelblonden, hellbraunen und dunkelbraunen Haares (Codes: Kerling 7/0, Fischbach & Miller 6923 und Kerling 2/0) von ca. 0,7 g Gewicht die 4-fache Menge der fertigen Anwendungsmischung appliziert. Nachdem die Strähnen für 30 min bei 32 °C blondiert wurden, wurden sie mit einem handelsüblichen Shampoo gewaschen und mit einem Föhn getrocknet.

### 2.3 Auswertung der Aufhelleistung

Jede Haarsträhne wurde vor und nach dem Bleichvorgang farbmetrisch vermessen. Als Maß für die Aufhellleistung der jeweiligen Rezeptur wurde der ΔL-Wert gemäß folgender Formel herangezogen:

$$\Delta L = L_{nachher} - L_{vorher}$$

$L_{nachher}$ = Helligkeit der Strähne nach dem Bleichen
$L_{vorher}$ = Helligkeit der Strähne vor dem Bleichen

Für jede Rezeptur und jeden Haartyp erfolgte eine Doppelbestimmung, aus den Einzelwerten wurde jeweils der Mittelwert gebildet. Je größer der ΔL-Wert, desto besser ist die Aufhellleistung der jeweiligen Rezeptur.

### 2.4 Aufhellleistung mit Aktivator 5

| Haartyp | ΔL (Rezeptur V1) | ΔL (Rezeptur E1) | ΔΔL |
|---|---|---|---|
| dunkelblond (Kerling 7/0) | 9,2 | **9,8** | **0,6** |
| hellbraun (Fischbach & Miller 6923) | 8,7 | **9,4** | **0,7** |
| dunkelbraun (Kerling 2/0) | 3,5 | **4,6** | **1,1** |

Die erfindungsgemäße Rezeptur E1 erzielt auf allen Haartypen deutlich verbesserte Aufhellleistungen.

## Patentansprüche

1. Mittel zum Aufhellen von keratinischen Fasern, **dadurch gekennzeichnet, dass** es in einem kosmetischen Träger

    (i) mindestens ein Oxidationsmittel, ausgewählt aus Wasserstoffperoxid und/oder einem seiner festen Anlagerungsprodukte an organische oder anorganische Verbindungen, und
    (ii) mindestens ein kationisches Phthalimid der Formel (I),

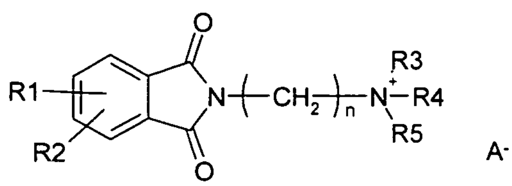

(I),

enthält, worin

R1 und R2 jeweils unabhängig voneinander für ein Wasserstoffatom, ein Halogenatom, eine Hydroxygruppe, eine $C_1$-$C_6$-Alkoxygruppe, stehen
oder R1 und R2 zusammen mit dem angrenzenden Benzolring eine weitere Phthalimid-Einheit der Formel (Ia) bilden,

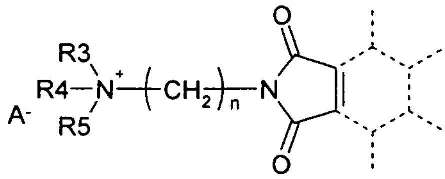
(Ia),

R3, R4, R5 jeweils unabhängig voneinander für eine $C_1$-$C_6$-Alkylgruppe, eine partiell oder vollständig halogenierte $C_1$-$C_6$-Alkylgruppe, eine $C_2$-$C_6$-Alkenylgruppe, eine $C_2$-$C_6$-Hydroxyalkylgruppe, eine $C_2$-$C_6$-Polyhydroxyalkylgruppe, eine $C_1$-$C_6$-Alkoxy-$C_2$-$C_6$-alkylgruppe, eine Cyano-$C_1$-$C_6$-aikylgruppe, eine Aryl-$C_1$-$C_6$-alkylgruppe, eine Carboxy-$C_1$-$C_6$-alkylgruppe oder eine Sulfonyl-$C_1$-$C_6$-alkylgruppe stehen oder
zwei der Substituenten R3, R4 und R5 zusammen mit dem angebundenen Stickstoff einen 5-, 6- oder 7-gliedrigen, gesättigten oder ungesättigten Ring bilden, der gegebenenfalls weitere Heteroatome enthält,
n für eine ganze Zahl von 1 bis 6 steht und
$A^-$ für ein physiologisch verträgliches Anion steht.

2. Mittel nach Anspruch 1, **dadurch gekennzeichnet, dass** es als Phthalimid gemäß Formel (I) mindestens eine Verbindung enthält, bei der die Substituenten R1 und R2 jeweils für ein Wasserstoffatom, stehen.

3. Mittel nach einem der Ansprüche 1 oder 2, **dadurch gekennzeichnet, dass** es als Phthalimid gemäß Formel (I) mindestens eine Verbindung enthält, bei die Substituenten R3, R4 und R5 jeweils unabhängig voneinander für eine $C_1$-$C_6$-Alkylgruppe, eine $C_2$-$C_6$-Alkenylgruppe oder eine $C_2$-$C_6$-Hydroxyalkylgruppe stehen.

4. Mittel nach einem der Ansprüche 1 bis 3, **dadurch gekennzeichnet, dass** es als Phthalimid gemäß Formel (I) mindestens eine Verbindung enthält, die ausgewählt ist aus der Gruppe von physiologisch verträglichen Salzen, deren kationische Anteile gebildet werden von Salzen des 2-(1,3-Dioxo-1,3-dihydro-2-isoindol-2-yl)-N,NN-trimethylethanaminiums, Salzen des 2-(1,3-Dioxo-1,3-dihydro-2H-isoindol-2-yl)-N,N-diethyl-N-methylethanaminiums, Salzen des N-[2-(1,3-Dioxo-1,3-dihydro-2H-isoindol-2-yl)ethyl]-N,N-diethylprop-2-en-1-aminiums, Salzen des N-Allyl-N-[2-(1,3-dioxo-1,3-dihydro-2H-isoindol-2-yl)ethyl]-N-methylprop-2-en-1-aminiums, Salzen des N-[2-(1,3-Dioxo-1,3-dihydro-2H-isoindol-2-yl)ethyl]-N-methyl-N-propylpropan-1-aminiums, Salzen des N-[2-(1,3-Dioxo-1,3-dihydro-2H-isoindol-2-yl)ethyl]-N,N-dipropylprop-2-en-1-aminiums, Salzen des 3-(1,3-Dioxo-1,3-dihydro-2H-isoindol-2-yl)-N,N,N-trimethyl-propan-1-aminiums, Salzen des N-[3-(1,3-Dioxo-1,3-dihydro-2H-isoindol-2-yl)propyl]-N,N-dimethylprop-2-en-1-aminiums, Salzen des 3-(1,3-Dioxo-1,3-dihydro-2H-isoindol-2-yl)-N,N-diethyl-N-methylpropan-1-aminiums und Salzen des N-[3-(1,3-Dioxo-1,3-dihydro-2H-isoindol-2-yl)propyl]-N,N-diethylprop-2-en-1-aminiums.

5. Mittel nach einem der Ansprüche 1 bis 4, **dadurch gekennzeichnet, dass** es als Phthalimid gemäß Formel (I) mindestens eine Verbindung aus 2-(1,3-Dioxo-1,3-dihydro-2H-isoindol-2-yl)-N,N-diethyl-N-methylethanaminium-p-toluol-sulfonat, N-Allyl-N-[2-(1,3-dioxo-1,3-dihydro-2H-isoindol-2-yl)ethyl]-N-methylprop-2-en-1-aminium-p-toluolsulfonat und N-[2-(1,3-Dioxo-1,3-dihydro-2H-isoindol-2-yl)ethyl]-N-methyl-N-propylpropan-1-aminium-p-toluolsulfonat enthält.

6. Mittel nach einem der Ansprüche 1 bis 5, **dadurch gekennzeichnet, dass** das oder die Phthalimid(e) der Formel (I) in einer Menge von 0,01 bis 25 Gew.-%, insbesondere von 0,1 bis 10 Gew.-%, jeweils bezogen das Gesamtgewicht des anwendungsbereiten Mittels, enthalten ist.

7. Mittel nach einem der Ansprüche 1 bis 6, **dadurch gekennzeichnet, dass** es als Oxidationsmittel Wasserstoffperoxid als wässrige Lösung enthält.

8. Mittel nach einem der Ansprüche 1 bis 7, **dadurch gekennzeichnet, dass** zusätzlich mindestens ein anorganisches

Persulfat- oder Peroxodisulfat-Salz, insbesondere Ammoniumperoxodisulfat, Kaliumperoxodisulfat und/oder Natriumperoxodisulfat, enthalten ist.

**9.** Kosmetische Verwendung eines Mittels nach einem der Ansprüche 1 bis 8 zum Aufhellen von keratinhalten Fasern, insbesondere menschlichen Haaren.

**10.** Mehrkomponentenverpackungseinheit (Kit-of-Parts) zum Aufhellen keratinischer Fasern, **dadurch gekennzeichnet, dass** sie in getrennt voneinander konfektionierten Containern mindestens eine Oxidationsmittelzubereitung (B), enthaltend Wasserstoffperoxid oder eine festen Anlagerungsverbindung von Wasserstoffperoxid an anorganische oder organische Verbindungen, und mindestens eine Zubereitung (A) enthält, wobei die Zubereitung (A) in einem kosmetischen Träger mindestens ein Phthalimid der Formel (I) enthält,

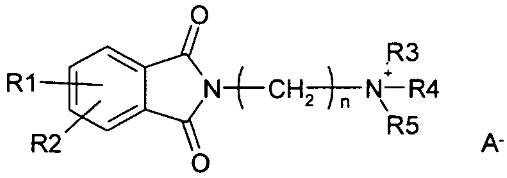

(I),

worin

R1 und R2 jeweils unabhängig voneinander für ein Wasserstoffatom, ein Halogenatom, eine Hydroxygruppe, eine $C_1$-$C_6$-Alkoxygruppe,
oder R1 und R2 zusammen mit dem angrenzenden Benzolring eine weitere Phthalimid-Einheit der Formel (Ia) bilden,

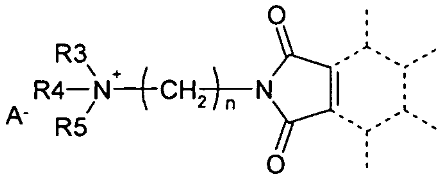

**(Ia),**

R3, R4, R5 jeweils unabhängig voneinander für eine $C_1$-$C_6$-Alkylgruppe, eine partiell oder vollständig halogenierte $C_1$-$C_6$-Alkylgruppe, eine $C_2$-$C_6$-Alkenylgruppe, eine $C_2$-$C_6$-Hydroxyalkylgruppe, eine $C_2$-$C_6$-Polyhydroxyalkylgruppe, eine $C_1$-$C_6$-Alkoxy-$C_2$-$C_6$-alkylgruppe, eine Cyano-$C_1$-$C_6$-alkylgruppe, eine Aryl-$C_1$-$C_6$-alkylgruppe, eine Carboxy-$C_1$-$C_6$-alkylgruppe oder eine Sulfonyl-$C_1$-$C_6$-alkylgruppe stehen oder
zwei der Substituenten R3, R4 und R5 zusammen mit dem angebundenen Stickstoff einen 5-, 6- oder 7-gliedrigen, gesättigten oder ungesättigten Ring bilden, der gegebenenfalls weitere Heteroatome enthält,
n für eine ganze Zahl von 1 bis 6 steht und
$A^-$ für ein physiologisch verträgliches Anion steht.

**Claims**

**1.** An agent for lightening keratinic fibres, **characterised in that** it contains in a cosmetic carrier

(i) at least one oxidising agent selected from hydrogen peroxide and/or one of the solid addition products thereof onto organic or inorganic compounds, and
(ii) at least one cationic phthalimide of formula (I),

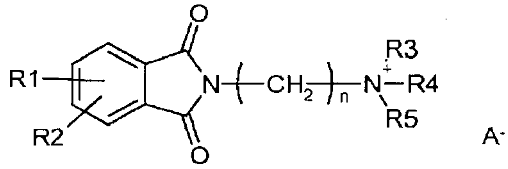

(I)

in which

R1 and R2 in each case mutually independently denote a hydrogen atom, a halogen atom, a hydroxyl group, a $C_1$-$C_6$ alkoxy group,
or R1 and R2 form, together with the adjacent benzene ring, a further phthalimide unit of formula (Ia),

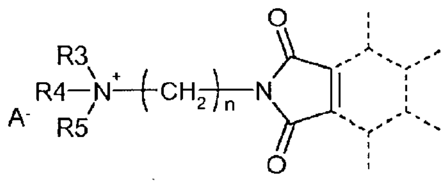

(Ia),

R3, R4, R5 in each case mutually independently denote a $C_1$-$C_6$ alkyl group, a partially or completely halogenated $C_1$-$C_6$ alkyl group, a $C_2$-$C_6$ alkenyl group, a $C_2$-$C_6$ hydroxyalkyl group, a $C_2$-$C_6$ polyhydroxyalkyl group, a $C_1$-$C_6$-alkoxy-$C_2$-$C_6$-alkyl group, a cyano-$C_1$-$C_6$-alkyl group, an aryl-$C_1$-$C_6$-alkyl group, a carboxy-$C_1$-$C_6$-alkyl group or a sulfonyl-$C_1$-$C_6$-alkyl group or
two the substituents R3, R4 and R5 form, together with the attached nitrogen, a 5-, 6- or 7-membered, saturated or unsaturated ring which optionally contains further heteroatoms,
n denotes an integer from 1 to 6 and
$A^-$ denotes a physiologically acceptable anion.

2. An agent according to claim 1, **characterised in that** it contains as the phthalimide according to formula (I) at least one compound in which the substituents R1 and R2 in each case denote a hydrogen atom.

3. An agent according to either one of claim 1 or claim 2, **characterised in that** it contains as the phthalimide according to formula (I) at least one compound in which the substituents R3, R4 and R5 in each case mutually independently denote a $C_1$-$C_6$ alkyl group, a $C_2$-$C_6$ alkenyl group, or a $C_2$-$C_6$ hydroxyalkyl group.

4. An agent according to any one of claims 1 to 3, **characterised in that** it contains as the phthalimide according to formula (I) at least one compound which is selected from the group of physiologically acceptable salts, the cationic moieties of which are formed of salts of 2-(1,3-dioxo-1,3-dihydro-2-isoindol-2-yl)-N,NN-trimethylethanaminium, salts of 2-(1,3-dioxo-1,3-dihydro-2H-isoindol-2-yl)-N,N-diethyl-N-methylethanaminium, salts of N-[2-(1,3-dioxo-1,3-dihydro-2H-isoindol-2-yl)ethyl]-N,N-diethylprop-2-en-1-aminium, salts of N-allyl-N-[2-(1,3-dioxo-1,3-dihydro-2H-isoindol-2-yl)ethyl]-N-methylprop-2-en-1-aminium, salts of N-[2-(1,3-dioxo-1,3-dihydro-2H-isoindol-2-yl)ethyl]-N-methyl-N-propylpropan-1-aminium, salts of N-[2-(1,3-dioxo-1,3-dihydro-2H-isoindol-2-yl)ethyl]-N,N-dipropylprop-2-en-1-aminium, salts of 3-(1,3-dioxo-1,3-dihydro-2H-isoindol-2-yl)-N,N,N-trimethylpropan-1-aminium, salts of N-[3-(1,3-dioxo-1,3-dihydro-2H-isoindol-2-yl)propyl]-N,N-dimethylprop-2-en-1-aminium, salts of 3-(1,3-dioxo-1,3-dihydro-2H-isoindol-2-yl)-N,N-diethyl-N-methylpropan-1-aminium and salts of N-[3-(1,3-dioxo-1,3-dihydro-2H-isoindol-2-yl)propyl]-N,N-diethylprop-2-en-1-aminium.

5. An agent according to any one of claims 1 to 4, **characterised in that** it contains as the phthalimide according to formula (I) at least one compound from among 2-(1,3-dioxo-1,3-dihydro-2H-isoindol-2-yl)-N,N-diethyl-N-methylethanaminium p-toluenesulfonate, N-allyl-N-[2-(1,3-dioxo-1,3-dihydro-2H-isoindol-2-yl)ethyl]-N-methylprop-2-en-1-aminium p-toluenesulfonate and N-[2-(1,3-dioxo-1,3-dihydro-2H-isoindol-2-yl)-ethyl]-N-methyl-N-propylpropan-1-aminium p-toluenesulfonate.

6. An agent according to any one of claims 1 to 5, **characterised in that** the phthalimide(s) of formula (I) is/are present in a quantity of 0.01 to 25 wt.%, in particular of 0.1 to 10 wt.%, in each case relative to the total weight of the ready-to-use agent.

7. An agent according to any one of claims 1 to 6, **characterised in that**, as oxidising agent, it contains hydrogen peroxide as an aqueous solution.

8. An agent according to any one of claims 1 to 7, **characterised in that** at least one inorganic persulfate or peroxodisulfate salt, in particular ammonium peroxodisulfate, potassium peroxodisulfate and/or sodium peroxodisulfate, is additionally present.

9. Cosmetic use of an agent according to any one of claims 1 to 8 for lightening keratin-containing fibres, in particular human hair.

10. A multicomponent packaging unit (kit of parts) for lightening keratinic fibres, **characterised in that** it contains in separately packaged containers at least one oxidising agent preparation (B) containing hydrogen peroxide or a solid addition compound of hydrogen peroxide on inorganic or organic compounds, and at least one preparation (A), wherein the preparation (A) contains in a cosmetic carrier at least one phthalimide of formula (I),

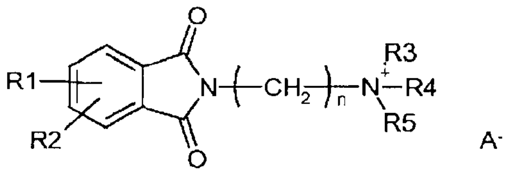

(I)

in which

R1 and R2 in each case mutually independently denote a hydrogen atom, a halogen atom, a hydroxyl group, a $C_1$-$C_6$ alkoxy group,
or R1 and R2 form, together with the adjacent benzene ring, a further phthalimide unit of formula (Ia),

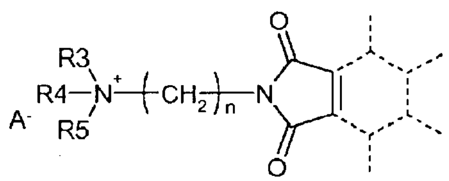

(Ia),

R3, R4, R5 in each case mutually independently denote a $C_1$-$C_6$ alkyl group, a partially or completely halogenated $C_1$-$C_6$ alkyl group, a $C_2$-$C_6$ alkenyl group, a $C_2$-$C_6$ hydroxyalkyl group, a $C_2$-$C_6$ polyhydroxyalkyl group, a $C_1$-$C_6$-alkoxy-$C_2$-$C_6$-alkyl group, a cyano-$C_1$-$C_6$-alkyl group, an aryl-$C_1$-$C_6$-alkyl group, a carboxy-$C_1$-$C_6$-alkyl group or a sulfonyl-$C_1$-$C_6$-alkyl group or
two the substituents R3, R4 and R5 form, together with the attached nitrogen, a 5-, 6- or 7-membered, saturated or unsaturated ring which optionally contains further heteroatoms,
n denotes an integer from 1 to 6 and
$A^-$ denotes a physiologically acceptable anion.

## Revendications

1. Agent pour la décoloration de fibres kératiniques, **caractérisé en ce qu'**il contient, dans un support cosmétique :

(i) au moins un agent d'oxydation choisi parmi le peroxyde d'hydrogène et/ou un de ses produits solides de fixation par addition sur des composés organiques ou inorganiques ; et
(ii) au moins un phtalimide cationique répondant à la formule (I)

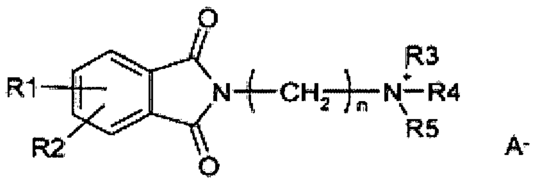

(I),

dans laquelle :

R1 et R2 représentent, chaque fois indépendamment l'un de l'autre, un atome d'hydrogène, un atome d'halogène, un groupe hydroxyle, un groupe alcoxy en $C_1$-$C_6$, ou bien R1 et R2 forment ensemble avec le noyau benzénique adjacent, une unité phtalimide supplémentaire répondant à la formule (Ia)

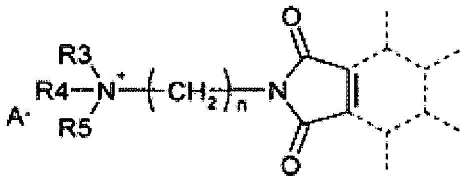

(Ia),

R3, R4, R5 représentent, chaque fois indépendamment l'un de l'autre, un groupe alkyle en $C_1$-$C_6$, un groupe alkyle en $C_1$-$C_6$ partiellement ou complètement halogéné, un groupe alcényle en $C_2$-$C_6$, un groupe hydroxyalkyle en $C_2$-$C_6$, un groupe polyhydroxyalkyle en $C_2$-$C_6$, un groupe alcoxy(en $C_1$-$C_6$)alkyle en $C_2$-$C_6$, un groupe cyanoalkyle en $C_1$-$C_6$, un groupe arylalkyle en $C_1$-$C_6$, un groupe carboxyalkyle en $C_1$-$C_6$ ou un groupe sulfonylalkyle en $C_1$-$C_6$, ou bien
deux des substituants R3, R4 et R5 forment ensemble avec l'atome d'azote auquel ils sont liés, un noyau saturé ou insaturé de 5, 6 ou 7 membres, qui contient de manière facultative d'autres hétéroatomes ;
n représente un nombre entier de 1 à 6 ; et
$A^-$ représente un anion physiologiquement acceptable.

2. Agent selon la revendication 1, **caractérisé en ce qu'**il contient, à titre de phtalimide répondant à la formule (I), au moins un composé dans lequel les substituants R1 et R2 représentent à chaque fois un atome d'hydrogène.

3. Agent selon l'une quelconque des revendications 1 ou 2, **caractérisé en ce qu'**il contient, à titre de phtalimide répondant à la formule (I), au moins un composé dans lequel les substituants R3, R4 et R5 représentent chaque fois, indépendamment l'un de l'autre, un groupe alkyle en $C_1$-$C_6$, un groupe alcényle en $C_2$-$C_6$ ou un groupe hydroxyalkyle en $C_2$-$C_6$.

4. Agent selon l'une quelconque des revendications 1 à 3, **caractérisé en ce qu'**il contient, à titre de phtalimide répondant à la formule (I), au moins un composé qui est choisi parmi le groupe de sels physiologiquement acceptables dont les fractions cationiques sont formées par des sels du 2-(1,3-dioxo-1,3-dihydro-2-isoindol-2-yl)-N,N,N-triméthyl-éthanaminium, des sels du 2-(1,3-dioxo-1,3-dihydro-2H-isoindol-2-yl)-N,N-diéthyl-N-méthyléthanaminium, des sels du N-[2-(1,3-dioxo-1,3-dihydro-2H-isoindol-2-yl)éthyl]-N,N-diéthylprop-2-én-1-aminium, des sels du N-allyl-N-[2-(1,3-dioxo-1,3-dihydro-2H-isoindol-2-yl)éthyl]-N-méthylprop-2-én-1-aminium, des sels du N-[2-(1,3-dioxo-1,3-dihydro-2H-isoindol-2-yl)éthyl]-N-méthyl-N-propylpropan-1-aminium, des sels du N-[2-(1,3-dioxo-1,3-dihydro-2H-isoindol-2-yl)éthyl]-N,N-dipropylprop-2-én-1-aminium, des sels du 3-(1,3-dioxo-1,3-dihydro-2H-isoindol-2-yl)-N,N,N-triméthyl-propan-1-aminium, des sels du N-[3-(1,3-dioxo-1,3-dihydro-2H-isoindol-2-yl)propyl]-N,N-diméthylprop-2-én-1-aminium, des sels du 3-(1,3-dioxo-1,3-dihydro-2H-isoindol-2-yl)-N,N-diéthyl-N-méthylpropan-1-aminium et des sels du N-[3-(1,3-dioxo-1,3-dihydro-2H-isoindol-2-yl)propyl]-N,N-diéthylprop-2-én-1-aminium.

5. Agent selon l'une quelconque des revendications 1 à 4, **caractérisé en ce qu'**il contient, à titre de phtalimide répondant à la formule (I), au moins un composé choisi parmi le p-toluènesulfonate de 2-(1,3-dioxo-1,3-dihydro-2H-isoindol-2-yl)-N,N-diéthyl-N-méthyléthanaminium, le p-toluène-sulfonate de N-allyl-N-[2-(1,3-dioxo-1,3-dihydro-2H-isoindol-2-yl)éthyl]-N-méthylprop-2-én-1-aminium et le p-toluène-sulfonate de N-[2-(1,3-dioxo-1,3-dihydro-2H-isoindol-2-yl)éthyl]-N-méthyl-N-propylpropan-1-aminium.

6. Agent selon l'une quelconque des revendications 1 à 5, **caractérisé en ce qu'**il contient le ou les phtalimide(s) répondant à la formule (I) en une quantité de 0,01 à 25 % en poids, en particulier de 0,1 à 10 % en poids, chaque fois rapportés au poids total de l'agent prêt à l'emploi.

7. Agent selon l'une quelconque des revendications 1 à 6, **caractérisé en ce qu'**il contient, à titre d'agent d'oxydation, du peroxyde d'hydrogène sous la forme d'une solution aqueuse.

8. Agent selon l'une quelconque des revendications 1 à 7, **caractérisé en ce qu'**il contient en outre au moins un sel inorganique de persulfate ou de peroxodisulfate, en particulier le peroxodisulfate d'ammonium, le peroxodisulfate de potassium et/ou le peroxodisulfate de sodium.

9. Utilisation cosmétique d'un agent selon l'une quelconque des revendications 1 à 8 pour la décoloration de fibres kératiniques, en particulier de cheveux humains.

10. Unité de conditionnement à plusieurs composants (kit-of-parts) pour la décoloration de fibres kératiniques, **caractérisée en ce qu'**elle contient dans des récipients confectionnés séparés les uns des autres, au moins une préparation d'agent d'oxydation (B) contenant du peroxyde d'hydrogène ou un composé solide de fixation par addition de peroxyde d'hydrogène à des composés inorganiques ou organiques, et au moins une préparation (A), la préparation (A) contenant dans un support cosmétique, au moins un phtalimide répondant à la formule (I)

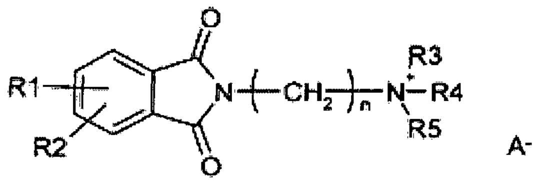

(I),

dans laquelle :

R1 et R2 représentent, chaque fois indépendamment l'un de l'autre, un atome d'hydrogène, un atome d'halogène, un groupe hydroxyle, un groupe alcoxy en $C_1$-$C_6$, ou bien R1 et R2 forment ensemble avec le noyau benzénique adjacent, une unité phtalimide supplémentaire répondant à la formule (Ia)

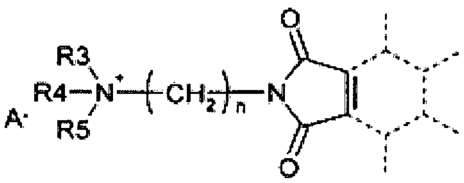

(Ia),

R3, R4, R5 représentent, chaque fois indépendamment l'un de l'autre, un groupe alkyle en $C_1$-$C_6$, un groupe alkyle en $C_1$-$C_6$ partiellement ou complètement halogéné, un groupe alcényle en $C_2$-$C_6$, un groupe hydroxyalkyle en $C_2$-$C_6$, un groupe polyhydroxyalkyle en $C_2$-$C_6$, un groupe alcoxy(en $C_1$-$C_6$)alkyle en $C_2$-$C_6$, un groupe cyanoalkyle en $C_1$-$C_6$, un groupe arylalkyle en $C_1$-$C_6$, un groupe carboxyalkyle en $C_1$-$C_6$ ou un groupe sulfonylalkyle en $C_1$-$C_6$, ou bien
deux des substituants R3, R4 et R5 forment ensemble avec l'atome d'azote auquel ils sont liés, un noyau saturé ou insaturé de 5, 6 ou 7 membres, qui contient de manière facultative d'autres hétéroatomes ;
n représente un nombre entier de 1 à 6 ; et
$A^-$ représente un anion physiologiquement acceptable.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 508623 A2
- EP 1161936 A
- DE 102005003412